# EUROPEAN PATENT SPECIFICATION

(11) **EP 4 076 180 B1**
(45) Date of publication and mention of the grant of the patent: **07.08.2024**
(21) Application number: 20838198.8
(22) Date of filing: 18.12.2020
(51) Int. Cl.: A61B 5/11, A61B 5/06, A61B 5/00

(54) **TREMOR STABILISATION APPARATUS**
VORRICHTUNG ZUR TREMORSTABILISIERUNG
APPAREIL DE STABILISATION DE TREMBLEMENTS

(30) Priority: 20.12.2019 GB 201919086
(43) Date of publication of application: 26.10.2022
(73) Proprietor: GyroGear Limited, London N1 8HL (GB)
(72) Inventor: ONG, Faii, London N1 8HL (GB); BAXTER, Elliott, London N1 8HL (GB); IBRAHIM, Youssef, London N1 8HL (GB); KNIGHT, Stephen, London N1 8HL (GB); KOH, Benjamin, London N1 8HL (GB); MCCABE, Gordon, London N1 8HL (GB); DE PANISSE, Paul, London N1 8HL (GB); TYLER, Stuart, London N1 8HL (GB)
(74) Representative: HGF
(86) International application number: PCT/GB2020/053269
(87) International publication number: WO 2021/123795

(56) References cited:
- WO-A1-2016/102958
- DAGNAES-HANSEN: "Magnetically suspended flywheel in gimbal mount - Nonlinear modelling and simulation", JOURNAL OF SOUND UND VIBRATION, vol. 432, 13 August 2018 (2018-08-13), pages 327 - 350, XP002802509, Retrieved from the Internet <URL:https://www.sciencedirect.com/science/article/pii/S0022460X18303948#fig1> [retrieved on 20210301]

## Description

### Background

The present invention relates to improvements in or relating to tremor stabilisation apparatus and methods, in particular to gyroscopic devices for use in stabilisation of tremors of parts of the body, both physiological and pathological, especially the hands.

Involuntary muscle tremors occur in a range of neurological conditions, notably degenerative conditions such as Parkinson's disease.

US5058571 describes an early proposal in which a battery-driven gyroscope is held against the back face of the hand by a strap. A gyroscope seeks to maintain the orientation of its spinning axis and resists any action that seeks to cause a change in that orientation. Thus the theory of using a gyroscope is that the onset of a muscle tremor causes a movement in the hand but the gyroscope acts against that movement, substantially cancelling out the tremor. WO2016/102958A1, the Applicant's earlier patent application, according to the preamble of claim 1, discloses a gyroscopic device for tremor stabilisation. The gyroscopic device includes a rotatable flywheel mounted to a gimbal that is in turn mounted to a turntable within a housing of the gyroscopic device. The gimbal permits precession of the flywheel, and the flywheel and gimbal can rotate on the turntable to match the direction of the tremor. Elastomeric dampers are provided to control precession of the flywheel.

### Summary

In accordance with the present invention there is provided tremor stabilisation apparatus according to claim 1.

Providing a precession axis that is fixed relative to the housing, as defined above, provide for a more compact device with fewer moving parts. In addition, gyroscope forces generated by the flywheel are efficiently transferred to the user's body part through the hinge between the gimbal and the housing, and through the housing to the user's body part.

In some examples, the housing and the gimbal are arranged to provide a maximum angle of precession about the precession axis from the equilibrium position of about 60 degrees or less, preferably about 30 degrees or less, more preferably about 10 degrees, most preferably about 5 degrees.

The housing may comprise a stop arranged to contact the gimbal when the gimbal is rotated to the maximum angle of precession. In examples, the stop comprises a seat arranged to support the biasing member.

Preferably, the biasing member is arranged between the gimbal and the housing, and the tremor stabilisation apparatus may further comprise an elastomeric damper disposed between the biasing member and the housing.

The biasing member is preferably configured to increase a biasing force provided by the biasing member as the angle of precession increases. For example, the biasing member may comprise a spring.

Preferably, the biasing member is arranged between the gimbal and the housing, and the biasing member may comprise a magnetic assembly. The magnetic assembly may comprise a first magnet attached to the gimbal, and a second magnet attached to the housing such that the first and second magnets repel each other.

A side of the housing may comprise a mount for attachment of the tremor stabilisation apparatus to the part of the user's body. The side of the housing may define a plane that is approximately parallel to a surface of the part of the user's body. In the equilibrium position an angle between the flywheel rotational axis and a normal line from the plane of the side of the housing may be between about +45 degrees and about -45 degrees, preferably about 0 degrees. In this way, the flywheel rotational axis is preferably normal to the surface of the part of the user's body, for example normal to the back of the user's hand.

In preferred example, the biasing member may comprise an adjustable force biasing member. The adjustable force biasing member may comprise an actuator for adjusting the biasing force provided by the adjustable force biasing member. The tremor stabilisation apparatus may further comprises a controller arranged to control the actuator to adjust the biasing force.

The biasing force provided by the adjustable force biasing member can be controlled to alter the responsiveness of the tremor stabilisation apparatus to tremors, and to alter the restorative force that the user experiences. Therefore, the biasing force can be controlled to provide a tremor stabilisation apparatus that can be used on a wide variety of users and the tremor stabilisation apparatus can be controlled to make it responsive to different tremors experienced by the same user.

The tremor stabilisation apparatus may further comprise a sensor arranged to detect a characteristic of a movement of the part of the user's body to which the tremor stabilisation apparatus is attached in use. Preferably, the controller may be configured to receive a signal from the sensor and control the biasing force of the adjustable force biasing member based on the detected characteristic.

In examples, the characteristic of a movement of the part of the user's body comprises a tremor characteristic of the part of the user's body, for example a tremor amplitude, a tremor frequency, and/or a tremor acceleration.

In some examples, the tremor stabilisation apparatus further comprises a sensor arranged to detect rotation of the flywheel about the precession axis. In these examples, the controller may be configured to receive a signal from the sensor and control the biasing force of the adjustable force biasing member based on the detected rotation of the flywheel about the precession axis.

Therefore, the tremor stabilisation apparatus can be controlled to respond to tremors experienced by the user to provide effective tremor stabilisation.

The characteristic of a movement of the part of the user's body may comprise a tremor characteristic of the part of the user's body, for example a tremor amplitude, a tremor frequency, and/or a tremor acceleration.

The gyroscopic forces generated by the flywheel are dependent at least in part on the rotational speed of the flywheel. Therefore, by controlling the flywheel rotational speed of the flywheel the tremor stabilisation apparatus can be configured for particular users, and/or can react to a user's tremors to provide effective tremor stabilisation.

In preferred examples, the prime mover controller may be configured to control an acceleration and/or deceleration of the flywheel based on the detected characteristic. Controlling the acceleration and/or deceleration of the flywheel can generate a torque moment that can act to stabilise a user's tremors.

In examples, the gimbal may comprise a mounting portion for attachment of the flywheel, and a hinge portion extending radially of the mounting portion to cooperate with a hinge seat of the housing to form the hinge defining the precession axis.

The gimbal may comprise a first hinge portion extending in a first radial direction with respect to the flywheel, and a second hinge portion extending a second radial direction with respect to the flywheel. The gimbal may further comprise an arm extending from the mounting portion in a radial direction with respect to the flywheel between the first hinge portion and the second hinge portion. The arm may be arranged to engage the biasing member.

The gimbal may comprise a first arm extending from the mounting portion in a radial direction with respect to the flywheel between the first hinge portion and the second hinge portion. The first arm may be arranged to engage a first biasing member. The gimbal may further comprise a second arm extending from the mounting portion in a radial direction with respect to the flywheel on an opposite side of the first and second hinge portions. The second arm may be arranged to engage a second biasing member.

The hinge portion of the gimbal may extend in a first radial direction with respect to the flywheel, and the gimbal may further comprise an arm extending from the mounting portion in an approximately opposite direction to the hinge portion. The arm may be arranged to engage the biasing member.

In some examples, one of the gimbal and the housing comprises a ball, and the other of the gimbal and the housing comprises a socket adapted to receive the ball. The ball is configured to rotate within the socket to define the precession axis. In this example, the socket may comprise a guide arranged to limit rotation of the ball within the socket to a single plane such that the precession axis is fixed relative to the housing.

In preferred example the flywheel may comprise a central disc portion and a circumferential skirt extending in an axial direction of the flywheel rotation axis, the circumferential skirt defining a recessed cavity. The circumferential skirt may comprise at least 50% of the total mass of the flywheel, preferably at least 75% of the total mass of the flywheel.

Preferably, the gimbal is at least partly nested in the recessed cavity of the flywheel. In some examples, the prime mover may be mounted to the gimbal, and the prime mover may be at least partly nested in the recessed cavity of the flywheel.

Therefore, the tremor stabilisation apparatus can be compact and low-profile, reducing the weight of the tremor stabilisation apparatus and making it comfortable and easy for the user to wear.

Preferably, the prime mover comprises an electric motor having one or more of:
an aspect ratio of a height dimension in an axial direction of the flywheel rotation
axis to a width dimension perpendicular to the height dimension of about 1 or
less; and/or
a brushless electric motor; and/or
a brushless DC motor; and/or
a DC motor comprising a diametrically-polarised permanent magnet rotor;
   and/or
a DC motor comprising slotless and/or coreless windings and/or
an axial flux configuration.

In some examples, the prime mover comprises a motor, and the motor preferably includes a motor housing and a motor mount. The motor housing may be mounted within the motor mount, and the motor housing may be generally axially cylindrical about an output shaft of the motor and includes a plurality of co-planar tabs extending radially therefrom and substantially uniformly spaced. Each tab may be located within a corresponding slot formed in a correspondingly cylindrical inner surface of the motor mount. Each slot may have a dimension greater than that of the corresponding tab in a circumferential direction. A biasing member may be provided between a corresponding face of each tab and an adjacent wall of the cylindrical surface of the motor mount.

Such an arrangement reduces the torque imparted on the user's body part during start up of the prime mover and the flywheel.

The prime mover preferably comprises an electric motor, preferably a DC motor, and the tremor stabilisation apparatus may comprise a controller configured to control the electric motor. The controller is preferably configured to brake the electric motor in pulses to reduce braking torque generated by the flywheel during braking. Braking the electric motor reduces the torque generated by the flywheel when the flywheel is slowed and/or stopped, which in turn reduces the torque imparted onto the user's body part.

The gyroscopic forces generated by the flywheel are dependent at least in part on the rotational speed of the flywheel. Therefore, by controlling the flywheel rotational speed of the flywheel the tremor stabilisation apparatus can be configured for particular users, and/or can react to a user's tremors to provide effective tremor stabilisation.

Preferably, detecting a characteristic of a movement of the part of the user's body comprises detecting a tremor characteristic, for example a tremor amplitude, a tremor frequency, and/or a tremor acceleration.

### Brief Description of the Drawings

The above and other aspects of the present invention will now be described in further detail, by way of example only, with reference to the accompanying figures, in which:
FIG. 1 shows tremor stabilisation apparatus, including a gyroscope device, worn on the hand of a user;
FIG. 2 shows the gyroscope device of the tremor stabilisation apparatus of FIG. 1;
FIGS. 3A and 3B show cross-sections of an example gyroscope device;
FIG. 4 shows a cross-section of another example gyroscope device;
FIGS. 5A and 5B show enlarged views of a biasing member arranged to control precession of the rotational flywheel assembly of the gyroscope device;
FIG. 6 shows an alternative biasing member comprising magnets;
FIG. 7 shows an example gyroscope device having a ball and socket arrangement for providing the precession axis;
FIG. 8 shows a gyroscope device attached to a hand of a user;
FIGS. 9A and 9B show an example gyroscope device having a turntable;
FIG. 10 shows a cross-section of a gyroscope device that comprises a controller and an adjustable force biasing member;
FIG. 11 illustrates a method of controlling precession of a gyroscope device;
FIG. 12 illustrates a method of controlling flywheel rotational speed of a gyroscope device;
FIG. 13 illustrates test results showing mean tremor amplitude reduction for gyroscope devices that generate different angular momentums;
FIGS. 14A, 14B, 15 and 16 show cross-sections of examples of flywheels and rotatable flywheel assemblies of a gyroscope device;
FIG. 17 shows an example of an integrated motor and flywheel;
FIGS. 18A and 18B show cross-sections of a gyroscope device having a decoupled motor and flywheel;
FIG. 19 shows an example motor and flywheel arrangement of a gyroscope device;
FIG. 20 schematically illustrates a motor control circuit of a gyroscope device;
FIG. 21 illustrates an example rotatable flywheel assembly;
FIG. 22 illustrates an example rotatable flywheel assembly having a bearing between the flywheel and the gimbal; and
FIG. 23 schematically illustrates a method of manufacturing the flywheel of FIG. 21.

### Detailed Description

A gyroscope is a device having a rotatable disc, for example a flywheel, which is rotatable about a flywheel rotation axis. As the flywheel rotates, the gyroscope will resist the action of an applied couple and tends to maintain a fixed orientation. If the gyroscope is rotationally displaced, angular momentum is conserved through nutation of the device about an axis which is mutually perpendicular to the flywheel rotation axis and the axis through which the device is displaced.

A gyroscope will exert a gyroscopic moment which is proportional in magnitude to the moment of inertia of the flywheel, the angular velocity of the flywheel and the angular velocity of nutation. The direction vector of the gyroscopic moment is proportional to the vector cross product of the angular velocity of the flywheel and the angular velocity of the nutation of the device.

The apparatus of the present invention comprises a gyroscope device having a rotatable gyroscope assembly and a housing. The rotatable gyroscope assembly comprises a rotatable flywheel that is rotatable about a flywheel rotation axis. The flywheel is mounted for precession about a precession axis such that displacement of the flywheel is restricted to rotation about the precession axis. In some examples, the flywheel is mounted to a gimbal which is hingedly attached to the housing to define the precession axis. In other examples, the gimbal is mounted to a turntable of the housing so that the precession axis can be rotated within the housing. The housing is attachable to a part of a user's body, for example a hand, and in use the flywheel rotates and a tremor of the user's body part causes displacement the flywheel and gimbal about the precession axis, generating a counter-rotational force that opposes the tremor, thereby acting to stabilise the tremor.

The apparatus of the present invention may include a plurality of gyroscope devices spaced about the part of the user's body. The plurality of gyroscope devices together apply a cumulative net gyroscopic moment to the body when the state of equilibrium of the body is perturbed, such as during a tremor or rotational displacement, but allows for the use of smaller gyroscopes, thereby spreading the mass of the gyroscopes across the body part making the device easier to wear and also reducing the bulk of the apparatus, thereby hindering dexterity and movement to a lesser degree.

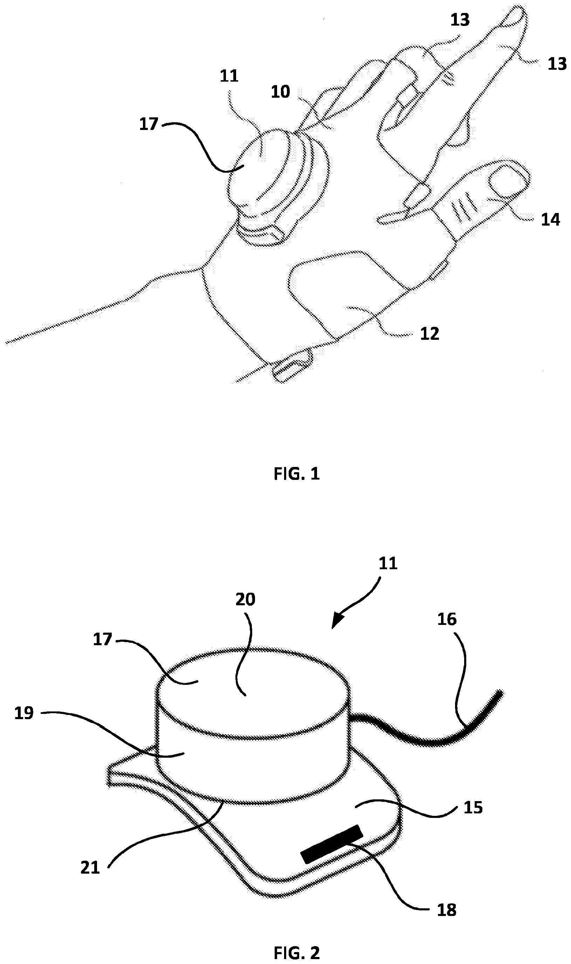

FIG. 1 shows an embodiment of a tremor stabilisation apparatus. The tremor stabilisation apparatus is a gyroscope device 11 that is attached to a glove 10 for a hand 12. In the embodiment shown, the glove 10 is of the open or fingerless type to allow free-movement of the fingers 13 and thumb 14. Preferably, the glove 10 is formed as a fabric support for the gyroscope device 11, attachable to the wrist, fingers and thumb of the wearer by means of straps, suitably straps using a hook and loop-type adjustable securing arrangement. The fabric is preferably of a soft, comfortable material that it can be worn comfortably for extended periods of time. In preferred embodiments, the fabric is of the type described in WO 2014/127291 in which van der Waals forces are developed between a soft silicone fabric surface and a wearer's skin, to retain the fabric in place.

In other examples, the glove 10 may be replaced with a simple strap or other means of attaching the gyroscope device 11 firmly to the hand or other part of the user's body. The attachment of the gyroscope device 11 to the user's body part is sufficiently rigid to transfer tremors from the body part to the gyroscope device 11, and to transfer gyroscopic forces from the gyroscope device 11 to the user's body part.

The described examples are of a gyroscope device 11 that can be attached to a user's hand 12, but it will be appreciated that the tremor stabilisation apparatus, in particular the gyroscope device 11, can be attached to any part of the user's body to stabilise tremors in that body part or in nearby body parts. For example, the gyroscope device 11 could be attached to a user's forearm, upper arm, shoulder, upper leg, lower leg, ankle, neck, torso, or head to stabilise tremors in those body parts. As described above, a user may be provided with a plurality of gyroscope devices 11 mounted to different body parts. The different gyroscope devices 11 may act to stabilise tremors in different body parts, or they may cooperate with each other to stabilise tremors in a particular body part. For example, a user may have a first gyroscope device 11 mounted to their upper arm, a second gyroscope device 11 mounted to their forearm, and a third gyroscope device 11 mounted to their hand, and all of the three gyroscope devices 11 would act to stabilise tremors in the user's arm and hand with the purpose of providing a steady hand for performing a task, such as eating.

FIG. 2 shows the gyroscope device 11 of a tremor stabilisation apparatus. The gyroscope device 11 has a mount 15 that is used to attach the gyroscope device 11 to the user's body, in this example a glove 10 and hand 12 as shown in FIG. 1. The gyroscope device 11 includes a cable 16 for providing power and/or control signals to the gyroscope device 11 from another component. For example, a power pack comprising a power source, such as a battery, may be attached to the user's arm, or elsewhere on the user's body, for example on a belt. The power pack may include a controller for controlling the gyroscope device 11 and connected by the cable 16, or the gyroscope device 11 may include a controller.

In examples, the power pack may be rechargeable by connecting it to a mains electricity supply, for example by a recharging cable. In examples the power pack has a single connector that can be connected either to a cable 16 of the gyroscope device 11 or to a recharging cable. In examples, the cable 16 of the gyroscope device 11 has a magnetic component and the connector of the power pack has an opposing magnetic component such that the magnetic components act to magnetically attract the cable 16 of the gyroscope device 11 to the connector. In examples, the power pack includes a sensor, for example a Hall effect sensor, configured to detect the magnetic component of the cable 16 of the gyroscope device 11. In this way, the power pack can detect if it is connected to the gyroscope device 11 or to a recharging cable (which doesn't have a magnetic component). In examples, a connector on the recharging cable that connects to the power pack comprises a shroud configured to prevent the recharging cable from being connected to the power pack when the power pack is being worn. For example, the shroud may comprise a protrusion arranged to surround a part of the power pack that is placed against the user, and therefore inaccessible, when the power pack is worn. Accordingly, the shroud can prevent the recharging cable from being connected to the power pack when the power pack is being worn. In some examples, the gyroscope device 11 has an integrated power supply, for example a battery, and in this example the cable 16 may not be needed.

As shown in FIGS. 1 and 2, the gyroscope device 11 includes a housing 17 that houses a rotatable flywheel assembly (not shown in FIG. 2). The housing 17 is generally cylindrical having a circumferential face 19 and opposing end faces 20, 21. In the illustrated examples the end faces 20, 21 of the housing 17 are planar, although in other examples one or both end faces 20, 21 may be curved, for example curved to match a contour of a user's body part to which it is attachable, for example the back of a hand 12.

As shown in FIG. 1 and 2, an end face 21 of the housing 17 is positioned on the back of the user's hand 12. In the illustrated example the gyroscope device 11 comprises a mount 15 in the form of a shaped plate 12 that is securable to the back of the hand 12 and/or to the glove 10 illustrated in FIG. 1 by means of straps (omitted for clarity) passing through apertures 18 formed in mount 15. Alternatively, the mount 15 may be mounted to the glove 10 by one or more fasteners, preferably a quick release fastener such as a bayonet fitting or clip or the like.

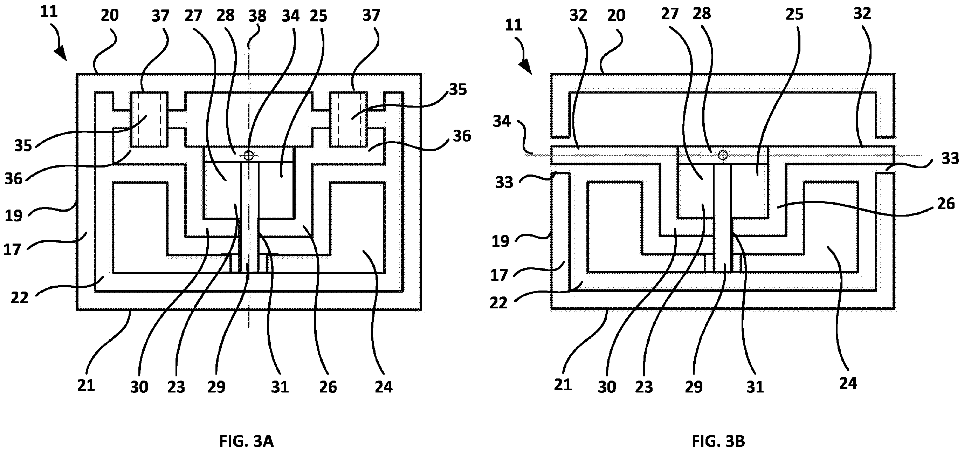

FIGS. 3A and 3B show cross-sectional views of an example gyroscope device 11 that has a fixed precession axis 34. The gyroscope device 11 comprises the housing 17 that is generally cylindrical and defines an interior cavity 22 in which a rotatable flywheel assembly 23 is housed. The rotatable flywheel assembly 23 comprises a flywheel 24, a motor 25 and a gimbal 26. The motor 25 includes a stator 27 and a rotor 28 including motor shaft 29. The flywheel 24 is mounted to the motor shaft 29. The flywheel 24 may be mounted to the motor shaft 29 by press fitting, by a keyed shaft arrangement, or by a fastener. The stator 27 of the motor 25 is attached to the gimbal 26, which, as shown in FIG. 3B, is pivotally mounted to the housing 17. The motor 25 is adapted to rotate the flywheel 24 about the flywheel rotation axis 38.

As illustrated, the gimbal 26 comprises a motor mounting portion 30 in the form of a planar member to which the motor 25 is attached. The motor mounting portion 30 is disposed between the motor 25 and the flywheel 24 and comprises an opening 31 through which the motor shaft 29 passes. The gimbal 26 also comprises hinge members 32 that extend beyond the outer edge of the flywheel 24 and cooperate with hinge seats 33 formed in the housing 17 to provide a hinge between the gimbal 26 and the housing 17. In this way, the rotatable flywheel assembly 23, in particular the gimbal 26, motor 25 and flywheel 24, are hingedly mounted within the housing 17 for rotation about precession axis 34. In FIG. 3B the precession axis 34 extends across the rotatable flywheel assembly 23, and in FIG. 3A the precession axis 34 is normal to the plane of the image. The hinge seats 33 and hinge members 32 provide a precession axis 34 that is fixed relative to the housing 17.

The motor 25 is thereby arranged to rotate the flywheel 24 within the housing 17, which is attached to the user's hand 12 as illustrated in FIG. 1.

As explained above, electrical power is provided via a cable (16, see FIG. 2) or from a battery within the housing 17. In some examples, an electrical connection is provided to the motor 25 by flexible wires that extend between a power terminal or battery in the housing 17 and the motor 25. The flexible wires accommodate movement of the motor 25 about the precession axis 34. Preferably, the flexible wires are arranged so that they do not twist or fold during precession of the rotatable flywheel assembly 23. The flexible wires may be routed from an opening in the housing 17 to the motor (and other electronic components) through one or more bends. In other examples, a slip ring is provided between the gimbal 26 and the housing 17 to provide an electrical connection to the motor 25. In other examples, an inductive coupling is provided to transfer electrical power from a power terminal or battery in the housing 17 to the motor 25, optionally via the gimbal 26.

When the user's hand 12 experiences a tremor the rotatable flywheel assembly 23 is angularly displaced about the precession axis 34. The gyroscopic effect of the rotating flywheel 24 generates a gyroscopic force that acts against the tremor. The gyroscopic force is transferred to the user's hand 12 through the housing 17 and mount 15. As explained further hereinafter, biasing members 35 are arranged to control precession of the rotatable flywheel assembly 23 about the precession axis 34.

As shown in FIG. 3A, the gimbal 26 further comprises plate members 36 that extend from the motor mounting portion 30 of the gimbal 26. The plate members 36 extend to a position where they oppose an internal surface 37 of the housing 17, with a space defined between. A biasing member, in this example a spring 35, is arranged between each plate member 36 and the internal surface 37 of the housing 17.

FIG. 3A shows a cross-section through the gyroscope device 11 that is at 90 degrees to the cross-section of FIG. 3B. In this example, the plate members 36 are angularly offset from the hinge members 32 about the flywheel rotation axis 38. Therefore, when a tremor causes the rotatable flywheel assembly 23 to rotate about the precession axis 34, as explained above, one of the plate members 36 acts to compress the associated spring 35. The force applied by the spring 35 on the housing 17 acts to urge the rotatable flywheel assembly 23 back to an equilibrium position (shown in FIGS. 3A and 3B).

In some examples, the springs 35 are attached to both the housing 17 and the plate members 36 of the gimbal 26 such that extension of the springs 35 also urges the rotatable flywheel assembly 23 back to an equilibrium position (shown in FIGS. 3A and 3B).

Therefore, the springs 35 are used to control precession of the rotatable flywheel assembly 23 about the precession axis 34. The biasing force provided by the springs 35 advantageously increases the frequency of tremors that can be stabilised by returning the rotatable flywheel assembly 23 to the equilibrium position more quickly than the rotatable flywheel assembly 23 would return of its own volition due to the gyroscopic force. As hand tremors typically have a small magnitude and high frequency (i.e. short and sharp tremors), the springs 35 advantageously allow the gyroscope device 11 to counteract successive tremors by limiting the angular displacement about the precession axis 34 and by returning the rotatable flywheel assembly 23 to the equilibrium position quickly.

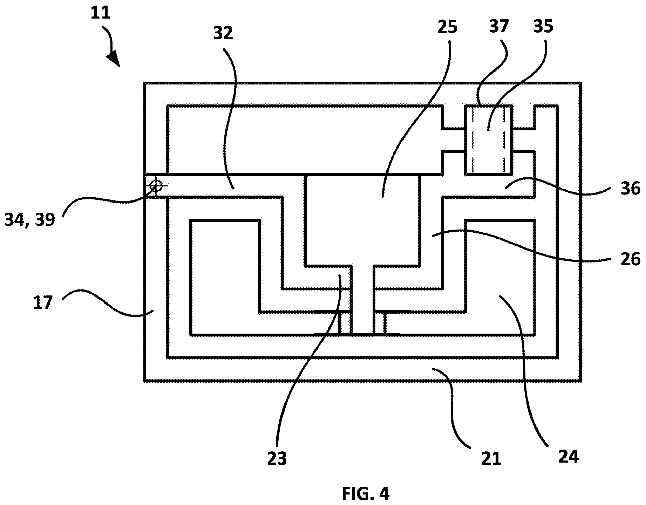

FIG. 4 illustrates an alternative gimbal 26 and spring 35 arrangement in which the gimbal 26 is pivotally mounted to the housing 17 about a hinge 39 formed on one side of the housing 17. A hinge member 32 of the gimbal 26 extends beyond the flywheel 24 to the hinge 39. In this example the hinge 39 defines the precession axis 34, which is fixed relative to the housing 17.

A plate member 36 of the gimbal 26 extends in an opposite direction to the hinge member 32 and engages a spring 35 in the same manner as described above. In this example, the spring 35 is attached to the internal surface 37 of the housing 17 and to the plate member 36 such that the spring 35 opposes precession of the rotatable flywheel assembly 23 in either direction about the precession axis 34, either through compression or extension of the spring 35.

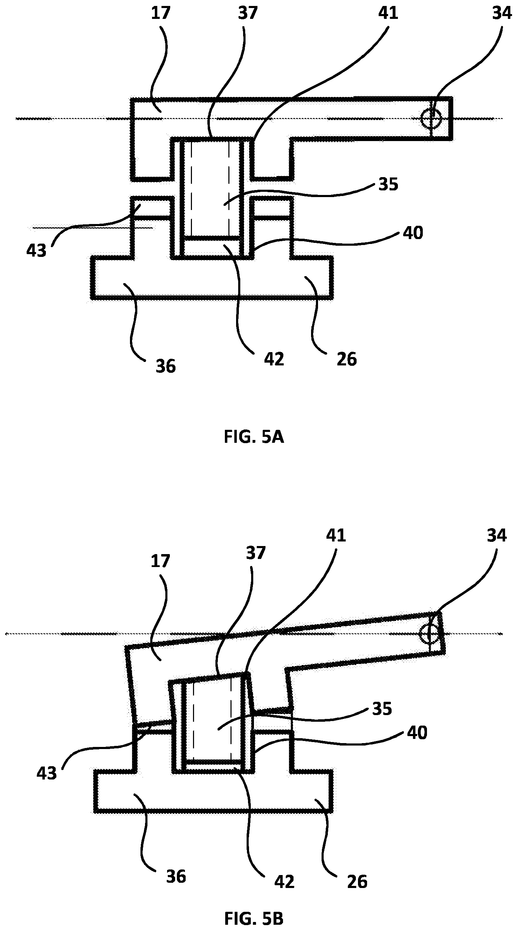

The housing 17 and the gimbal 26 are configured to limit rotation of the rotatable flywheel assembly 23 about the precession axis 34. FIG. 5A and 5B show enlarged views of the plate member 36, spring 35 and housing 17. FIG. 5A shows the plate member 36 in an equilibrium position. The plate member 36 includes a seat 40 for retaining a first end of the spring 35, and the internal surface 37 of the housing 17 includes a similar seat 41 for retaining the other end of the spring 35. As explained above, the spring 35 may be attached to the plate member 36 and/or the housing 17, in particular at the seats 40, 41.

In examples, an elastomeric dampener 42 is disposed between the spring 35 and the plate member 36. The elastomeric dampener 42 acts to dampen forces applied to the plate member 36 by the spring 35, and vice versa. The elastomeric dampener 42 may be, for example, a silicon or nylon insert. In some examples, the elastomeric dampener 42 is alternatively disposed between the housing 17 and the spring 35, in seat 41. In some examples, a first elastomeric dampener is provided between the spring 35 and the plate member 36, and second elastomeric dampener is provided between the housing 17 and the spring 35.

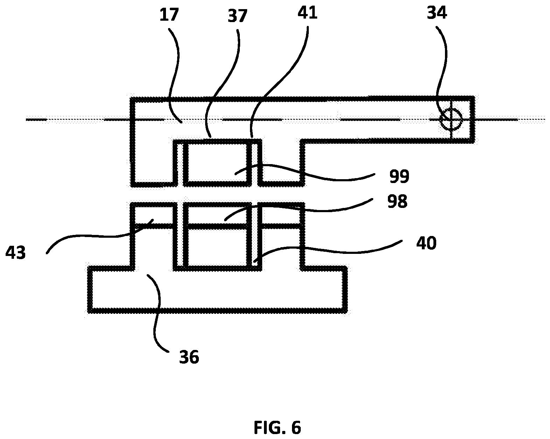

In the example of FIG. 6, the biasing member comprises a first magnet 98 attached to the gimbal 26, in particular in the seat 40 described with reference to FIGS. 5A and 5B, and a second magnet 99 attached to the housing 17, in particular in the seat 41 described with reference to FIGS. 5A and 5B. The magnets 98, 99 are arranged to repel each other, thereby providing a biasing force that opposes precession of the rotatable flywheel assembly 23.

As illustrated in FIGS. 5B and 6, a wall 43 of the plate member 36 may serve as a hard stop against precession of the rotatable flywheel assembly 23. In this example, at a maximum precession angle the wall 43 contacts the housing 17 and prevents further rotation. In alternative examples the housing 17 may comprise a wall that acts as a hard stop, in addition to or instead of the wall 43 illustrated.

In this way, the gimbal 26 and housing 17 are configured to limit rotation of the rotatable flywheel assembly 23 about the precession axis 34. In examples, the maximum angle of precession is preferably less than about 30 degrees, more preferably less than about 20 degrees, and more preferably about 10 degrees, and most preferably about 5 degrees. Limiting the angle of precession advantageously means that the rotatable gyroscope assembly 23 does not process more than is needed to generate a restorative force for a tremor, limits the magnitude of angular momentum that is generated to prevent the gyroscopic forces becoming too large, and ensures that the rotatable flywheel assembly 23 returns to the equilibrium position in a short amount of time such that any subsequent tremor can be counteracted (i.e. ensures that the gyroscope device 11 is reactive to successive tremors). Moreover, limiting the angle of precession provides for a more compact gyroscope device 11 because the housing 17 does not need to accommodate further rotation of the rotatable flywheel assembly 23 about the precession axis 34.

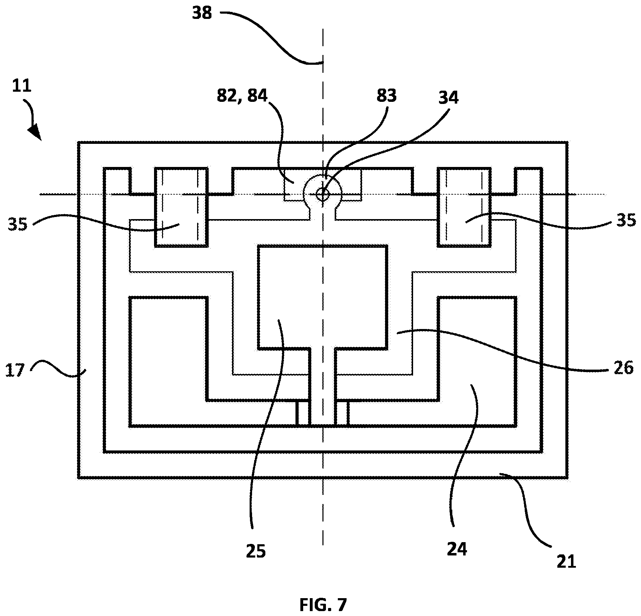

In the example gyroscope device 11 of FIG. 7 the gimbal 26 is mounted to the housing 17 via a ball and socket hinge 82 that defines the precession axis 34. The motor 25 and flywheel 24 are mounted to the gimbal 26, and as shown the gimbal 26 comprises a ball 83 and the housing 17 comprises a socket 84 that receives the ball 83 and allows the ball 83 and the gimbal 26 to rotate. The socket 84 is preferably shaped such that the ball 83 and the gimbal 26 can only rotate in one plane (the plane of the page as illustrated), or there are additional guides provided to limit rotation of the ball 83 and gimbal 26 to a single plane. This provides a hinge 82 with a fixed precession axis 34. As with the examples of FIGS. 3A to 6, one or more biasing members 35 are provided to act against rotation of the gimbal 26 about the precession axis 34. The ball and socket hinge 82 is advantageously disposed in line with the rotational axis 38 of the flywheel 24, and so the radial dimension of the rotatable flywheel assembly 23 is less than with the examples of FIGS. 3A to 6.

The biasing member or members 35 of the example of FIG. 7 may be provided in a seat, with a stop and an elastomeric dampener as illustrated in FIGS. 5A to 6.

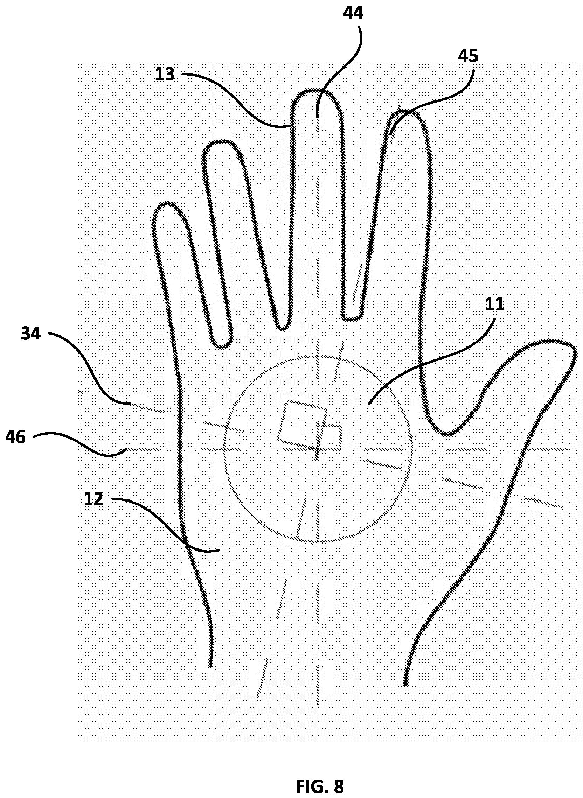

As explained above, the axis of precession 34 of the rotatable flywheel assembly 23 is defined by a hinge formed between the gimbal 26 and the housing 17. The orientation of the precession axis 34 is fixed with respect to the housing 17, and as explained previously the housing 17 is fixed with respect to the hand 12 of a user during use. FIG. 8 illustrates the gyroscope device 11 in position on the back of a user's hand 12. Axis 44 is an imaginary longitudinal axis of the hand 12 extending from the user's arm parallel to the usual position of the user's fingers 13 through the centre of the gyroscope device 11. As show, the precession axis 34 of the rotatable flywheel assembly 23 of the gyroscope device 11 defines a non-parallel, non-perpendicular angle with the hand axis 44.

As explained below, the angular offset between the precession axis 34 and the hand axis 44 allows tremors in the user's hand 12 to cause displacement of the rotatable flywheel assembly 23 about the precession axis 34, and also allows the gyroscopic force generated by the rotatable flywheel assembly 23 to counteract the tremor.

In particular, a tremor of a user's hand 12 will comprise some combination of rotation about the hand axis 44, a cross-hand axis 46 perpendicular to the hand axis 44 and in the plane of the hand 12, and a third axis (not shown) that is perpendicular to both the hand axis 44 and the cross-hand axis 46 (i.e. normal to the plane of the image in FIG. 7). Typically, the largest and most disruptive components of a hand tremor are rotation about the hand axis 44 and the cross-hand axis 46. The arrangement of the precession axis 34 shown in FIG. 7 provides for stabilisation of tremors about the hand axis 44 and the cross-hand axis 46 because rotation about either of these axes would cause precession of the rotatable flywheel assembly 23. Any angular offset between the third axis (not shown) and the flywheel rotation axis would also cause precession of the rotatable flywheel assembly 23 and therefore be stabilised by the gyroscope device 11.

In preferred examples, the angular offset between the precession axis 34 and the hand axis 44 is between 5 degrees and 85 degrees, preferably between 5 degrees and 45 degrees, more preferably between 10 degrees and 20 degrees. The preferred angular offset between the precession axis 34 and the hand axis 44 provides for greater stabilisation of tremors about the hand axis 44 than about the cross-hand axis 46 because tremors about the hand axis 44 are generally the most disruptive to tasks being performed.

Specifically, the gyroscopic effect generated by the angular momentum of the flywheel 24 acts at 90 degrees to the precession axis 34. Therefore, in the arrangement shown in FIG. 8 the gyroscope device 11 is orientated to primarily stabilise tremors of the hand 12 in the form of rotations about the hand axis 44. A tremor comprising a rotation about the hand axis 44 will displace the rotatable flywheel assembly 23 about the precession axis 34, resulting in a stabilisation force acting about axis 45 illustrated in FIG. 8, which is at 90 degrees to the precession axis 34. Due to the angular arrangement of the precession axis 34 with respect to the hand axis 44 a majority of the stabilisation force acts against the hand tremor about hand axis 44. In addition, due to the angular arrangement of the precession axis 34 with respect to the hand axis 44 a proportion of the stabilisation force also stabilises tremors of the hand 12 about the axis 46, which is perpendicular to the hand axis 44. Therefore, advantageously, the position of the precession axis 34 within the gyroscope device 11 can be fixed while still providing stabilisation of different tremors.

The angular arrangement of the precession axis 34 with respect to the hand axis 44 can be tailored for the tremor profile of a particular user. In the applicant's earlier application WO2016/102958A1 the rotatable flywheel assembly is mounted to a turntable within the housing so that the angular offset is varied according to the tremor. However, the inventors have found that the angular position of the precession axis with respect to the user's hand 12 can be fixed, which advantageously provides effective tremor stabilisation while maintaining a small, low profile and lighter gyroscope device 11 with fewer moving parts. In addition, a fixed precession axis, as described above, improves transfer for the gyroscopic forces from the gyroscope device 11 to the user's hand 12 because there are fewer moving parts between the flywheel 24 and the mount 15, thereby reducing any damping that might be provided by such moving parts (e.g. due to flex, play in bearings, or the like).

In some examples, the position of the precession axis 34 with respect to the user's hand 12 can be set based on a tremor profile of the user. For example, a user who primarily experiences hand tremors about hand axis 44 could be provided with a gyroscope device 11 having a precession axis 34 that is aligned with the hand axis 44 so that the stabilisation force is only provided about the hand axis 44. However, most users will experience a tremor profile that is best addressed by an angular offset of between 5 degrees and 85 degrees between the hand axis 44 and the precession axis 34, as shown in FIG. 8. In particular, an angular offset of between 5 degrees and 45 degrees, or between 20 and 30 degrees, will provide effective tremor stabilisation for most user tremor profiles.

In some examples, the gyroscope device 11 is configured such that the precession axis 34 is parallel to the cross-hand axis 46 or the hand axis 44. As explained above, a user's hand tremor comprises movement in different directions and so it is possible for the rotatable flywheel assembly 23 to be angularly displaced (i.e. precess) in any orientation of the precession axis 34 on the hand 12. In addition, if the gyroscope device 11 is used on other body parts then the precession axis 34 can be arranged in different orientations according to the tremors of that body part.

In addition, the spring or springs 35 provided to control precession of the rotatable flywheel assembly 23 and to return the rotatable flywheel assembly 23 to the equilibrium position can be selected according to a user's tremor profile. In particular, a user with higher magnitude, lower frequency tremors would be best addressed by springs 35 having a lower spring rate than a user with lower magnitude, higher frequency tremors. Therefore, the springs 35 can be selected to provide a customised gyroscope device 11.

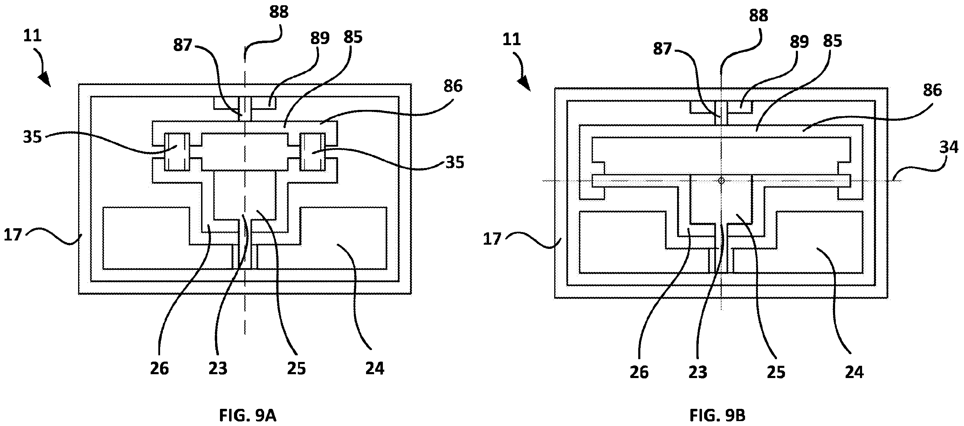

In the example of FIGS. 9A and 9B, the housing 17 comprises a turntable assembly 85 for mounting the gimbal 26. In this example, the precession axis 34 can be rotated within the housing 17 such that the orientation of the precession axis 34 with respect to the user' body part can change after the gyroscope device 11 has been attached to the user's body part. In this example, as illustrated, the housing 17 comprises a turntable assembly 85 having a turntable 86 to which the gimbal 26 is pivotally mounted in a similar manner as the gimbal 26 of FIGS. 3A and 3B is mounted to the housing 17. In particular, the rotatable flywheel assembly 23 (i.e. gimbal 26, motor 25, and flywheel 24) is hingedly mounted to the turntable so that the rotatable flywheel assembly 23 can rotate about a precession axis 34 defined between the turntable 86 and the gimbal 26. Biasing members, for example springs 35, are arranged to act between the turntable 86 and the gimbal 26. In this way, the biasing members 35 act between the gimbal 26 and the housing 17, via the turntable assembly 85. The turntable 86 is mounted to the housing 17 via pivot 87 that defines a rotational axis 88 for rotation of the turntable 86 and with it, the rotatable flywheel assembly 23 of the gimbal 26, motor 25, and flywheel 24.

In some examples, a motor 89 may be provided to control rotation of the turntable 86 and rotatable flywheel assembly 23, or the turntable 86 and rotatable flywheel assembly 23 may be freely rotatable about the pivot 87 within the housing 17 so that the rotatable flywheel assembly 23 can orientate itself based on a user's tremors.

In preferred examples, the biasing member 35 acting between the gimbal 26 and the housing 17 or turntable 86 of the gyroscope device 11 comprises an adjustable force biasing member. An adjustable force biasing member, as described above, may be provided to any of the example gyroscope devices 11 of FIGS. 3A to 9.

For example, the adjustable force biasing member may comprise an adjustable spring, for example a compression spring having a threaded shaft extending through the middle of a compression spring and a threaded adjusting nut mounted on the threaded shaft such that rotation of the threaded shaft and/or the threaded adjusting nut compresses or extends the compression spring, changing the biasing force that it provides. An actuator may be provided to rotate the threaded shaft and/or the adjusting nut.

In another example, the adjustable force biasing member may comprise an adjustable force gas spring wherein a pressure of gas within the adjustable force gas spring can be varied to control the biasing force provided by the adjustable force gas spring. An actuator may be provided to reduce or increase gas pressure in the adjustable force gas spring. The actuator may comprise a release valve for reducing pressure and/or a compressor for increasing pressure.

In a further example, the adjustable force biasing member may comprise an electromagnet arrangement in which an electromagnet is provided in the housing 17 or turntable 86, and an opposing permanent magnet is provided on the gimbal (or vice versa). In this arrangement, controlling the electrical power provided to the electromagnet controls the biasing force provided by the adjustable force biasing member. An actuator may be provided to control the electromagnet.

In some examples, the biasing force of the adjustable force biasing member may be set to configure the gyroscope device 11 for a particular user. Specifically, the biasing force can be set to control precession of the rotatable flywheel assembly 23 in a manner that is customised to a user's requirements, as discussed above. For example, the biasing force and/or maximum precession angle can be set based on a user's tremor amplitude and frequency. A user with lower magnitude, higher frequency tremors would be provided with a higher biasing force and smaller maximum precession angle, while a user with higher magnitude, lower frequency tremors would be provided with a lower biasing force and a higher maximum precession angle.

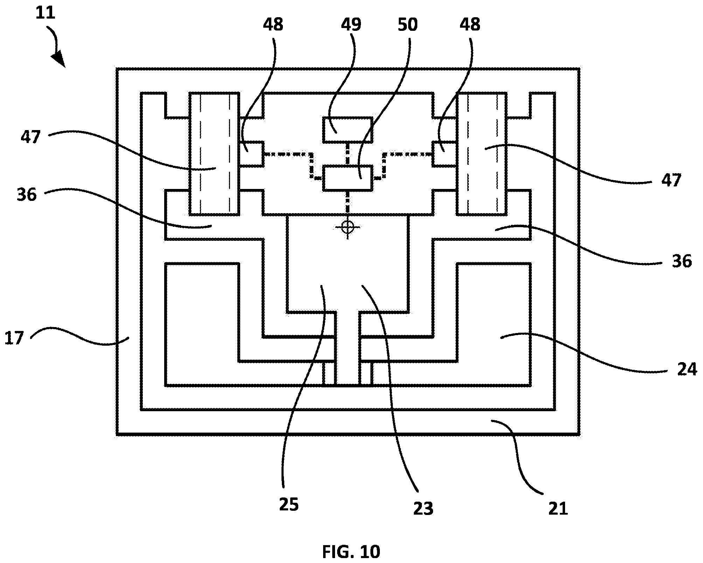

In other examples, the gyroscope device 11 may be configured to adjust the biasing force of the adjustable force biasing member during operation, that is, dynamically. This allows the biasing force to be varied according to a user's current tremors. In addition, this arrangement advantageously means that a single device could be configured for different user's based on their specific tremors. FIG. 10 is a schematic illustration of a gyroscope device 11 having a dynamic control system for controlling the adjustable force biasing member dynamically according to a detected tremor. The illustrated example is based on the example of FIGS. 3A and 3B but it will be appreciated that it can also be applied to the examples of FIGS. 4, 7, and 9A and 9B.

FIG. 10 illustrates the gyroscope device 11 having a housing 17 and a rotatable flywheel assembly 23. The rotatable flywheel assembly 23 includes a flywheel 24, motor 25 and gimbal 26. The gimbal 26 is rotatably mounted to the housing 17 in the same manner as described with reference to FIGS. 3A and 3B. In this example, adjustable force biasing members 47 are provided between the housing 17 and the plate members 36 of the gimbal 26. Each adjustable force biasing member 47 has an actuator 48 for changing the biasing force provided by the adjustable force biasing member 47.

The gyroscope device 11 of FIG. 10 also includes a sensor 49 arranged to detect a movement of a user's hand to which the gyroscope device 11 is attached, for example a tremor. In the illustrated example, the sensor 49 is attached to the housing 17. However, the sensor 49 may be located elsewhere in the gyroscope device 11, or may be located outside of the housing 17, for example directly on the hand or arm of the user. The sensor 49 is preferably an accelerometer arranged to detect a movement of the hand, for example a tremor. The sensor 49 preferably detects hand rotations (tremors) about at least two axes, in particular the hand axis 44 and the cross-hand axis 46 illustrated in FIG. 8. The sensor 49 detects one or more characteristics of the movement of the hand 12, for example one or more tremor characteristics. For example, the accelerometer may detect any one or more of amplitude, frequency, and/or acceleration of tremors, such as hand tremors.

As shown in FIG. 10, the gyroscope device 11 further includes a controller 50 that is arranged to receive signals from the sensor 49. The controller 50 is configured to control the actuators 48 of the adjustable force biasing members 47 based on the detected tremors.

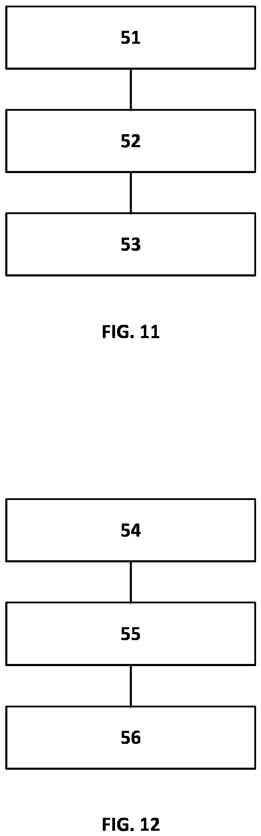

As illustrated in FIG. 11, in a method of controlling the gyroscope device 11 the controller 50 is configured to receive a sensor signal from the sensor 49, 51. This may comprise receiving movement characteristic data (e.g. tremor amplitude, frequency, acceleration) for the detected movement of the part of the user's body, or it may comprise receiving an unprocessed signal and determining the characteristic(s) of the movement (e.g. tremor amplitude, frequency, acceleration).

The controller is further configured to determine a target biasing force for the adjustable force biasing member 47, 52. The target biasing force is based on the movement characteristic(s). The controller 50 is further configured to control the actuators 48 of the adjustable force biasing members 47 to provide the target biasing force, 53. The target biasing force may be based on the detected movement characteristic(s). The controller 50 may comprise a memory storing a table of target biasing forces according to the detected movement characteristic(s). The controller 50 may retrieve a target biasing force from the memory based on the detected movement characteristic(s) and control the adjustable force biasing members 47 to provide the target biasing force.

In alternative examples, the controller 50 controls the actuators 48 of the adjustable force biasing members 47 according to a proportional relationship between the detected movement characteristic(s) and a configuration of the actuator. The proportional relationship may be defined in the controller. Therefore, the controller 50 does not need to determine or retrieve an actual target biasing force value when controlling the adjustable force biasing members 47 based on the detected movement characteristic(s).

In this way, a gyroscope device 11 can be mounted to any user and it will configure operation of the adjustable force biasing members according to the user's movements, for example the user's tremors. In addition, such a gyroscope device 11 can effectively counteract a user's tremors when those tremors vary in magnitude and frequency, as is common in people affected by Parkinson's and Essential Tremors.

Additionally or alternatively, the gyroscope device 11 may comprise a sensor (not shown) arranged to detect rotation of the rotatable flywheel assembly 23 about the precession axis 34. Such a sensor may detect a precession angle relative to an equilibrium position in which the rotatable flywheel assembly 23 is positioned when there is no movement of the user's hand. For example, the sensor may comprise a rotary position sensor. In other examples, the sensor is arranged to detect a power being drawn by the motor 25, particularly an electrical current being drawn by the motor 25. When the rotatable flywheel assembly 23 rotates about the precession axis 34 it has been found that the gyroscopic forces applied to the motor shaft result in a higher power being drawn to rotate the flywheel 24. Therefore, precession of the rotatable flywheel assembly 23 can be detected by sensing the power drawn by the motor 25. Alternatively or additionally, the sensor may be arranged to detect a rotational speed of the flywheel 24. In particular, the flywheel 24 rotational speed will be reduced by precession of the rotatable flywheel assembly 23 due to the gyroscopic forces acting on the motor 25, which increases friction in the motor. The sensor may be arranged to detect an actual rotational speed of the flywheel 24 and determine a rotational speed error in comparison to the speed that the motor 25 should be rotating at (according to the controller). This rotational speed error will be proportional to the angle of precession of the rotatable flywheel assembly 23 and so can be used to detect precession of the rotatable flywheel assembly 23.

In this example, the controller 50 may receive a signal from the sensor and control the actuator 48 to adjust the biasing force of the adjustable force biasing member 47 based on the detected angle of precession. For example, if the sensor detects a higher angle of precession the controller 50 may increase the biasing force provided by the adjustable force biasing member 47. In this way, the biasing force provided by the adjustable force biasing member 47 can be controlled based on the amount of precession of the rotatable flywheel assembly 23, which is at least partly determined by the acceleration and magnitude of any hand movements, specifically tremors. Therefore, detecting the angle of precession allows the biasing force provided by the adjustable force biasing members 47 to be appropriate to the user's movements. In addition, the adjustable force biasing members 47 can be controlled to prevent the rotatable flywheel assembly 23 from grounding out, i.e. contacting the stop 43 described with reference to FIGS. 5A and 5B, which may damage the flywheel 24 and/or motor 25.

Such a method is also illustrated in FIG. 11, in which the controller 50 is configured to receive a sensor signal from the sensor 49, 51 indicating an angle of rotation about the precession axis 34.

The controller is further configured to determine a target biasing force for the adjustable force biasing member 47, 52 based on the detected angle of precession. The controller 50 is further configured to control the actuators 48 of the adjustable force biasing members 47 to provide the target biasing force, 53. The controller 50 may comprise a memory storing a table of target biasing forces according to the detected angle of precession. The controller 50 may retrieve a target biasing force from the memory based on the detected angle of precession and control the adjustable force biasing members 47 to provide the target biasing force.

In alternative examples, the controller 50 controls the actuators 48 of the adjustable force biasing members 47 according to a proportional relationship between the detected angle of precession and a configuration of the actuator. The proportional relationship may be defined in the controller. Therefore, the controller 50 does not need to determine or retrieve an actual target biasing force value when controlling the adjustable force biasing members 47 based on the detected angle of precession.

As explained previously, the force generated by the gyroscope device 11 to stabilise the user's tremors is based primarily on the angular momentum generated by the rotating flywheel 24, and on the displacement torque applied to the flywheel 24 by a user's tremor (i.e. precession). Therefore, the gyroscope device 11 will generate a higher counteracting gyroscopic force in response to a stronger tremor (and vice versa) even if the rotational speed of the flywheel 24 is steady.

Although the magnitude of the gyroscopic force generated by the flywheel 24 is inherently dependent on the severity of the tremor (i.e. the displacement torque applied to the flywheel 24 about the precession axis 34), as described below the gyroscope device 11 may additionally or alternatively be configured to control the rotational speed of the flywheel 24 to control the angular momentum generated by the gyroscope device 11. In this way, the range of forces provided by the gyroscope device 11 can be customised for a particular user with particular movement characteristics, for example tremor characteristics.

Angular momentum is a function of the inertia and rotational speed of the flywheel 24. inertia is a function of mass and diameter of the flywheel 24, including how the mass is distributed through the radius of the flywheel 24.

A gyroscope device 11 for use on a user's body part, for example a hand, is preferably of a size and weight that does not inhibit voluntary movements of the body part and allows the user to comfortably wear the gyroscope device 11, for example as illustrated in FIG. 1.

In particular, for use on a user's hand the gyroscope device 11 preferably has a maximum weight of about 1 kg and a maximum dimension across the gyroscope device 11 of about 80 mm. In the illustrated examples the housing 17 of the gyroscope device 11 is cylindrical to accommodate the cylindrical flywheel 24. Therefore, in examples the maximum diameter of the housing 17 is preferably about 80 mm. Preferably, for use on a user's hand, the maximum weight of the gyroscope device 11 is about 0.5 kg, and the maximum diameter of the gyroscope device 11 is about 60 mm. Such a gyroscope device 11 is comfortable for a user to wear on their hand 12 as shown in FIG. 1.

For use on other body parts it will be appreciated that the gyroscope device 11 may be larger and heavier. For example, for use on a user's arm or leg the maximum weight of the gyroscope device 11 may be about 2 kg, more preferably about 1 kg, and the maximum diameter may be about 180 mm, more preferably about 100 mm to 150 mm. Stronger, heavier limbs such as arms and legs will require higher gyroscopic forces to stabilise stronger tremors, and so the flywheels 24 for use on these body parts are preferably heavier, for example up to 1 kg, and larger, for example up to about 160 mm.

Within the size and weight constraints described above, a gyroscope device 11 that is designed to be worn by a user can be customised for a particular user by selecting a flywheel 24 that, at a given rotational speed of the flywheel 24, provides an appropriate amount of force to stabilise tremors in the body part to which the gyroscope device 11 is attached. The force generated by the gyroscope device 11 is preferably a balance between providing enough force to stabilise tremors while still permitting voluntary movements of the body part and providing a gyroscope device 11 that is comfortable for a user to wear.

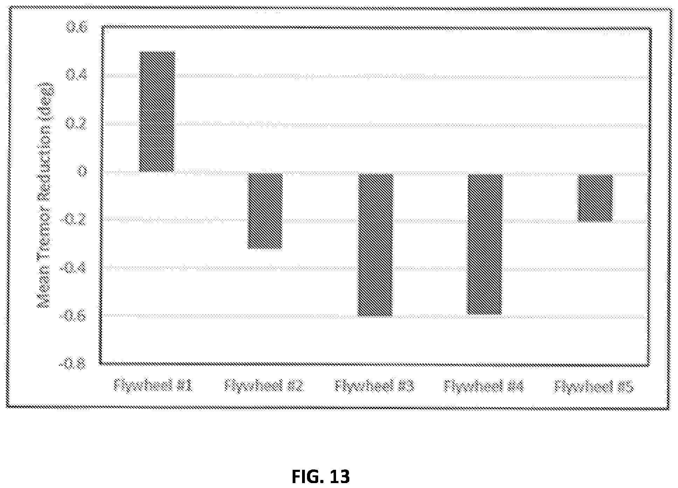

The inventors have found that some ranges of angular momentum are particularly effective at hand tremor stabilisation for a gyroscope device 11 for use on a user's hand. In particular, as illustrated in the test results shown in FIG. 13, the inventors have shown that an angular momentum in the range of about 0.05 kgm²/s to 0.30 kgm²/s, more particularly in the range of about 0.08 kgm²/s to 0.2 kgm²/s, provides effective hand tremor stabilisation for a wide range of users while still allowing the users to make voluntary hand movements to perform tasks.

In particular, tests showed that for most users an angular momentum in the range of 0.05 kgm²/s to 0.30 kgm²/s provided most effective hand tremor stabilisation without inhibiting voluntary hand movements. Angular momentum below this range was found to be ineffective at stabilising hand tremors, while angular momentum greater than this range caused suppression of voluntary hand movements, generated gyroscopic forces that were too large so that additional tremors were imparted to the user, and/or made the gyroscope device 11 too heavy and large to be worn on a user's hand.

The tests described below were conducted on 46 subjects overall. 14 of the subjects have been diagnosed with Parkinson's Disease, and 32 of the subjects have been diagnosed with Essential Tremor. All of the tests were conducted on the same hand for each subject, typically but not exclusively the subject's dominant hand. All subjects were above 18 years old.

The subjects were provided with five different gyroscope devices worn on the user's hand. The specifications of the flywheel of each of the different gyroscope devices are detailed in the below table.

**Table 1 - Flywheel Specifications for Tests**

| | **Flywheel #1** | **Flywheel #2** | **Flywheel #3** | **Flywheel #4** | **Flywheel #5** |
|---|---|---|---|---|---|
| **Flywheel Mass (kg)** | 0.0047 | 0.195 | 0.152 | 0.195 | 0.152 |
| **Flywheel Diameter (mm)** | 14 | 52 | 51 | 52 | 51 |
| **Flywheel Inertia (kgm²)** | 1.0E-6 | 6.7E-5 | 5.9E-5 | 6.7E-5 | 5.9E-5 |
| **Rotational Speed (RPM)** | 14000 | 12,000 | 14,000 | 24,000 | 28,000 |
| **Angular Momentum (kg.m²/s)** | 0.002 | 0.084 | 0.086 | 0.168 | 0.173 |

An inertial measurement unit was attached to the hand of each subject during the tests. The inertial measurement unit is a Bosch BNO055 9-axis absolute orientation sensor. The inertial measurement unit was arranged to measure the hand Euler angle about three axes (x, y, z), hand rotational velocity about the three axes (x, y, z), and hand linear acceleration in direction of the three axes (x, y, z).

During the tests, the data output from the inertial measurement unit was used to determine average rotational hand tremor amplitude by combining the Euler angle data for all three axes as a vector sum and then calculating a mean average rotational hand tremor amplitude.

The subjects were asked to perform two activities, as detailed below:
1. Volumetric test - the subjects were asked to hold a 100ml beaker of water (filled) over a basin while sitting with their arm unsupported for 60 seconds. This activity was repeated 5 times for each test.
2. Eating test - the subjects were asked to transfer spoonful's of soybeans from a first bowl (75% full) to a second bowl (initially empty) located one bowl diameter apart from the first bowl. This activity was repeated 5 times for each test.

For each gyroscope device each subject was first asked to complete the activity with the gyroscope device switched off (i.e. no flywheel rotation). A baseline average rotational hand tremor amplitude is determined for each gyroscope device. Subsequently, for each gyroscope device each subject was asked to complete the activities as detailed above with the gyroscope device activated (i.e. with the flywheel rotating) and the average rotational hand tremor amplitude was measured.

FIG. 13 illustrates, for each of the five gyroscope devices detailed above, the mean reduction in rotational hand tremor amplitude (degrees). Specifically, FIG. 13 shows the mean difference in hand tremor amplitude between the baseline average rotational hand tremor amplitude for each gyroscope device and the average hand tremor amplitude during the activities with the gyroscope devices activated. The averages are taken across all of the tests conducted, i.e. across all of the test subjects and across all of the volumetric and eating activities.

As illustrated in the test results shown in FIG. 13, Flywheel #1, with an angular momentum of 0.002 kgm²/s resulted in an increase in mean tremor amplitude (degrees). Such an increase is attributable to the test subject having a weight attached to their hand, which made it more difficult for them to steady their hand, while the flywheel provided very little gyroscopic force and so provided only minimal tremor stabilisation. It was found that an angular momentum of at least about 0.05 kgm²/s was required to demonstrate a reduction in mean tremor amplitude.

Flywheels #2, #3 and #4 demonstrated effective tremor stabilisation, while flywheel #5 reduced tremor magnitude by less than flywheels #2, #3, and #4. It was found that an angular momentum greater than about 0.30 kgm²/s resulted in poor tremor reduction because the strength of the gyroscopic forces was apparently too great for the test subjects to control, resulting in additional tremors caused by the gyroscope device 11. In addition, it was found that an angular momentum greater than about 0.30 kgm²/s tended to suppress voluntary movements of the test subjects, meaning that the test subjects had to work harderto perform the tasks, which in turn reduced the effectiveness at tremor stabilisation.

Therefore, the test results demonstrate the effectiveness of a gyroscope device at stabilising a user's hand tremors and also demonstrate that angular momentum can be set or controlled to provide effective tremor stabilisation.

In particular, the test results indicate a preferred range of angular momentum for stabilising hand tremors of between about 0.05 kgm²/s and about 0.30 kgm²/s, more particularly between about 0.08 kgm²/s and 0.20 kgm²/s. Such a range has been shown to provide effective hand tremor stabilisation while still allowing the subjects to make voluntary hand movements to perform tasks.

In particular, the inventors have found that a gyroscope device 11 having a flywheel having a mass of about 0.150 kilograms and a diameter of about 50 millimetres, with an inertia of about 6×10⁻⁵kgm², can be operated at rotational speeds of between 8000 RPM and 50000 RPM to provide angular momentum in the range of about 0.05 kgm²/s to 0.30 kgm²/s that can provide tremor stabilisation to a wide range of tremors in a user's hand. Such a gyroscope device 11 would also be effective at stabilising tremors in other body parts that experience similar tremors, for example a user's forearm. Therefore, a gyroscope device 11 having such a flywheel can be used for a wide variety of users and the rotational speed of the flywheel can be configured for each user to provide an appropriate angular momentum within the range of about 0.05 kgm²/s to about 0.30 kgm²/s.

For other body parts, for example arms, legs, neck, back, head, the inventors found that larger angular momentum was required due to the higher strength of muscles in these areas (leading to stronger tremors) and the larger mass of the body part experiencing the tremors.

In some examples, the controller 50 illustrated in FIG. 10 is additionally or alternatively configured to control the motor 25 and the rotational speed of the flywheel 24. Therefore, the controller 50 can be configured to control the angular momentum of the flywheel 24 and the gyroscopic force provided to stabilise tremors. In these examples, the controller 50 may be configurable when setting up the gyroscope device 11 for a user to provide an appropriate angular momentum, and/or the controller 50 can be configured to dynamically control the rotational speed of the flywheel 24 based on a tremor characteristic or characteristics detected by the sensor49. Control of the rotational speed of the flywheel 24 may be provided in a gyroscope device 11 that includes passive biasing members, for example the springs 35 described with reference to FIGS. 3A to 5, 7, or 9A and 9B, or adjustable force biasing members as previously described with reference to FIG. 10.

For example, as illustrated in the method of controlling a gyroscope device 11 shown in FIG. 12, the controller 50 may be configured to receive a sensor signal from the sensor 49, 54. The sensor 49 may be arranged to detect a characteristic of a movement of the user's hand, for example a tremor characteristic, or an angle of precession, as described with reference to FIGS. 10 and 11.

The controller is further configured to determine a target angular momentum and/or a target rotational speed for the flywheel 24, 55. The target angular momentum and/or a target rotational speed is based on the sensor signals, for example the movement characteristic(s) and/or the angle of precession. The controller 50 is further configured to control the motor 25 to provide the angular momentum and/or a target rotational speed, 56.

The target angular momentum and/or a target rotational speed may be based on the detected movement characteristic(s) and/or angle of precession. The controller 50 may comprise a memory storing a table of target angular momentums and/or a target rotational speeds according to the detected movement characteristic(s) and/or angle of precession. The controller 50 may retrieve a target angular momentum and/or a target rotational speed from the memory based on the detected movement characteristic(s) and/or angle of precession, and control the motor 25 to provide the angular momentum and/or a target rotational speed. In some examples, the memory stores flywheel rotational speeds mapped against one or more movement characteristics and/or angles of precession, and the controller 50 retrieves a target flywheel rotational speed based on the detected movement characteristic(s) and/or angle of precession. In other examples, the memory stores target angular momentums mapped against one or more movement characteristics and/or angles of precession, and the controller 50 retrieves a target angular momentum based on the detected movement characteristic(s) and/or angle of precession, and then determines the target rotational speed of the flywheel 24 that corresponds to that target angular momentum. In this way, the same memory items (i.e. target angular momentums) can be used for different flywheels 24, i.e. flywheels 24 having different mass and/or radial mass distribution (rotational inertia).

In alternative examples, the controller 50 controls the motor 25 according to a proportional relationship between the detected movement characteristic(s) and a power and/or speed of the motor 25. The proportional relationship may be defined in the controller. Therefore, the controller 50 does not need to determine or retrieve an actual target angular momentum value or rotational speed value when controlling the motor 25 based on the detected movement characteristic(s).

In this way, a gyroscope device 11 can be mounted to any user and it will tune the motor 25 to provide an appropriate angular momentum for the user's movements, in particular the user's tremors. In addition, such a gyroscope device 11 can effectively counteract a user's movements, specifically tremors, when those tremors vary in magnitude and frequency, which is common in people affected by Parkinson's and Essential Tremors. Moreover, by dynamically controlling the rotational speed of the flywheel 24 the gyroscope device 11 can save energy and lengthen the operational life of the gyroscope device 11 by turning off the motor 25 when the user is not experiencing tremors.

In preferred examples, the controller 50 is configured to control the one or more adjustable force biasing members 47 to provide a target biasing force for precession, as described with reference to FIG. 11, and the controller is also configured to control the rotational speed of the flywheel 24 to provide a target angular momentum and/or flywheel rotational speed, as described with reference to FIG. 12. In this example, the gyroscope device 11 is dynamically operated to control flywheel angular momentum and the precession force based on a movement characteristic(s) and/or angle of precession as detected by the sensor 49 or sensors.

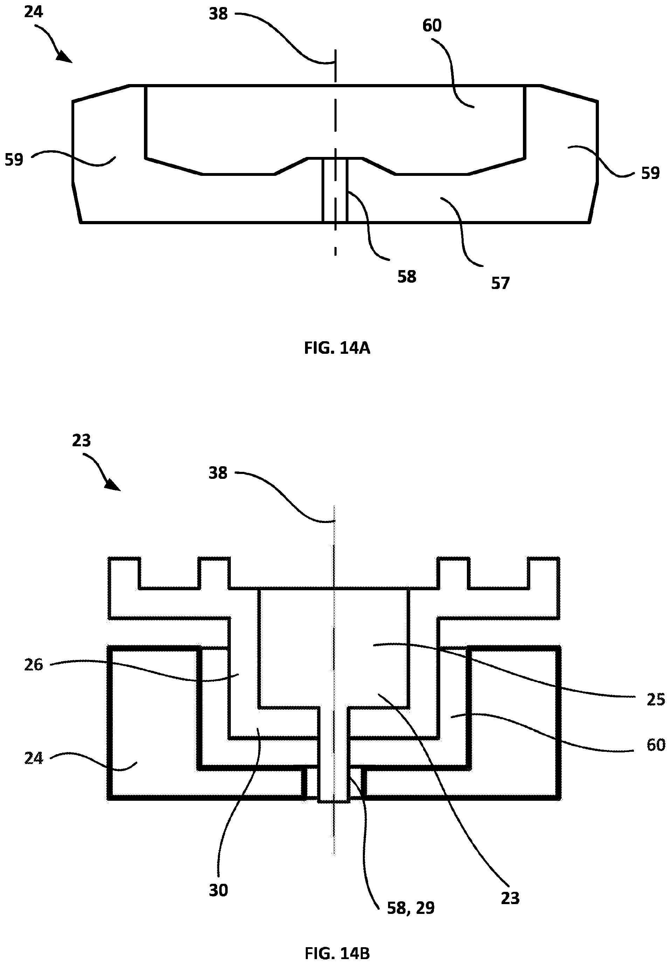

FIGS. 14A to 15 illustrate examples of the flywheel 24 for use in the gyroscope device 11. FIG. 14A shows the flywheel 24 in isolation, and FIG. 14B shows a cross-section of the rotatable flywheel assembly 23 including the flywheel 24. The flywheel is generally cylindrical about the flywheel rotational axis 38. As illustrated, the flywheel 24 comprises a central disc portion 57 comprising a hole 58 for attachment to the motor shaft 29. The central disc portion 57 is generally planar and is relatively thin. The flywheel 24 also comprises a circumferential skirt 59 extending from a circumferential edge of the central disc portion 57 in an axial direction of the flywheel rotational axis 38.

The flywheel 24 comprises a profile that provides a mass distribution focussed on the outer circumferential edge of the flywheel 24, i.e. at the circumferential skirt. That is, the circumferential skirt 59 comprises the majority of the total mass of the flywheel 24.

In preferred examples, the circumferential skirt 59 comprises at least 50% of the total mass of the flywheel 21, preferably at least 60% of the total mass of the flywheel 24, more preferably at least 75% of the total mass of the flywheel 24. The configuration of the circumferential skirt 59 concentrates mass at the circumferential edge of the flywheel 24, as illustrated, which provides a flywheel 24 with higher angular inertia that can generate the desired angular momentum while limiting the overall mass of the flywheel 24.

As the mass and diameter of the flywheel 24 determine the inertia and angular momentum of the flywheel 24, and also the outer dimensions of the gyroscope device 11, it is beneficial for the mass and diameter of the flywheel 24 to be appropriate for a gyroscope device 11 for attachment to a body part of a user, for example a hand of a user. Therefore, for a gyroscope device 11 for use on a user's hand the mass of the flywheel 24 is preferably between about 0.05 kg and about 0.5 kg, more preferably between about 0.1 kg and 0.2 kg. Preferably, the diameter of the flywheel 24 is less than about 150mm, preferably less than about 100mm, preferably less than about 80mm, preferably about 50mm.

For a gyroscope device 11 for use on a different body part, for example an arm or leg, the desired angular momentum is greater and the user can support a heavier gyroscope device 11. In such applications, the flywheel may have a mass of up to about 2kg, more preferably up to about 1kg, more preferably less than about 0.5kg, or between 0.2kg and 0.5kg. Similarly, a gyroscope device 11 for an arm or leg can be larger, and so the flywheel 24 diameter may be up to about 200 mm, more preferably about 150 mm.

Within these mass and diameter constraints the inventors have found that a flywheel with at least 75% of the mass in the circumferential skirt 59 can provide the desired range of angular momentum at rotational speeds varying between about 5000 RPM to 70000 RPM, more preferably between about 10000 RPM and 30000 RPM, more preferably between about 15000 RPM and 30000 RPM.

In addition, the circumferential skirt 59 of the flywheel 24 provides a recessed cavity 60 on one side of the flywheel 24. As illustrated in FIG. 14B, in preferred examples the gimbal 26 and the motor 25 are at least partly nested in the recessed cavity 60 of the flywheel 24. This advantageously provides a rotatable flywheel assembly 23 having a low profile, and helps to keep the centre of mass of the rotatable flywheel assembly 23, and the gyroscope device 11, closer to the surface of the user's body part during use. This advantageously reduces any effects of the weight of the gyroscope device 11, such as a torque generated by the weight of the gyroscope device 11 when the hand is rotated.

As shown in FIG. 14B, the gimbal 26 is dish-shaped, with the motor mounting portion 30 being disposed in the recessed cavity 60 between the flywheel 24 and the motor 25. This provides a motor 25 mounting position that is nested in the recessed cavity 60. The motor 25 is a low profile motor 25, as described hereinafter, configured to fit substantially within the recessed cavity 60 of the flywheel 24. In this way, the housing 17 can be closely matched to the size of the flywheel 24, which minimises the overall dimensions of the gyroscope device 11.

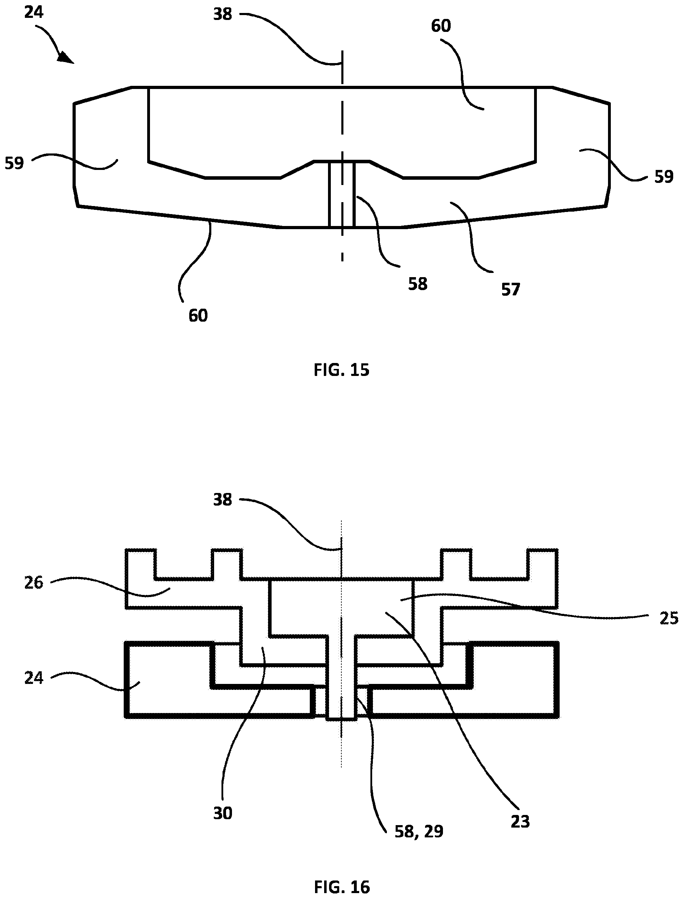

In the example of FIG. 16 the flywheel 24 has a lower profile, with a more even radial mass distribution than the flywheel of FIGS. 14A to 15, and with a lower proportion of the mass being at the circumferential skirt. All other factors being the same, the flywheel of FIG. 16 has a lower inertia and would generate less angular momentum and therefore lower gyroscopic forces for a given rotational speed. Such a flywheel may be used for user's with weaker tremors, or where the overall weight of the gyroscope device should be minimised, for example for children or elderly people. The flywheel 24 of this example could be rotated at higher speeds to achieve the same angular momentum as other flywheels for lower overall weight. As the angular momentum is the primary driver of the magnitude of the gyroscopic forces, such a lightweight device could be used to stabilise tremors while maintaining a low weight gyroscope device 11.

In addition, as illustrated in FIGS. 3A, 3B, 4, 7, 10, in a preferred arrangement the flywheel 24 is disposed adjacent to the side 21 of the housing 17 that is arranged against, or closest to, the user's body part during use. In this arrangement, the gimbal 26 and the motor 25 are arranged on the opposite side of the flywheel 24 to the user's body part. Such an arrangement is beneficial because the flywheel 24 is the heaviest part of the gyroscope device 11 and so arranging the flywheel 24 closer to the user's body part limits torque generated by the weight of the gyroscope device 11 on the user's body part, making the gyroscope device 11 more comfortable to wear. In addition, the gyroscopic forces of the gyroscope device 11 are more effective when they are closer to the axis of movement of the tremor, i.e. closer to the body part. Therefore, such an arrangement provides a gyroscope device 11 that is more comfortable for a user to wear and also more effective at tremor stabilisation.

In some examples, as illustrated in FIG. 15, a face 60 of the flywheel 24 opposite to the recessed cavity 60 is angled to accommodate rotation of the rotatable flywheel assembly 23 about the precession axis 34. In particular, the angle of the face 60 may match the angle of maximum rotation about the precession axis 34. This allows the flywheel 24 to be located closer to the side 21 of the housing 17, providing a lower profile gyroscope device 11 and the centre of mass of the gyroscope device 11 is closer to the user's body part.

In other examples, illustrated in FIGS. 3A, 3B, 4, 7, 10, 14A, 14B the face 60 of the flywheel 24 that is opposite to the recessed cavity 60 is planar, i.e. flat, or convex as illustrated in FIG. 15. As explained hereinafter, such a flywheel 24 is advantageous for manufacturing, i.e. machining, a balanced flywheel 24.

In preferred examples, the motor 25 is an electric motor, for example a brushless DC motor. A brushless DC motor is preferable to a brushed motor as it will generate less dust and other matter that may impede operation of the gyroscope device 11, for example by accumulating in bearings or on the flywheel 24. As described with reference to FIGS. 3A and 3B, the motor 25 comprises a stator 27 and rotor 28.

In preferred examples, the motor body, excluding the motor shaft 29, comprises an aspect ratio (ratio of a dimension in the axial direction of the axis of rotation 38 to a dimension in the radial direction) of about 1 or less, preferably about 0.5. This provides a low profile motor 25 that can nest in the recessed cavity 60 of the flywheel 24, as illustrated.

In preferred examples, the rotor 28 of the motor 25 comprises a diametrically-polarised magnet rotor. Such a rotor 28 provides for a low profile motor 25.

In preferred examples, the motor 25 comprises slotless and/or coreless windings, which provide for a compact and low profile motor 25.

In preferred example, the motor 25 comprises an axial flux arrangement, which provides for a compact and low profile motor 25 that can be more closely nested in the recessed cavity 60 of the flywheel 24, as illustrated.

In other examples, the motor 25 is replaced by an alternative prime mover arranged to rotate the flywheel 24. For example, the prime mover may comprise a pneumatic motor driven by compressed air supplied from a compressor. The compressor may be carried on the user's body, or it may be part of an external source. For example, if the tremor stabilisation apparatus were used at a workstation (e.g. in a factory) to assist the user, then compressed air can be provided from an external compressor via a hose. For portable tremor stabilisation apparatus (i.e. carried with the user wherever they go), the prime mover is preferably an electric motor.

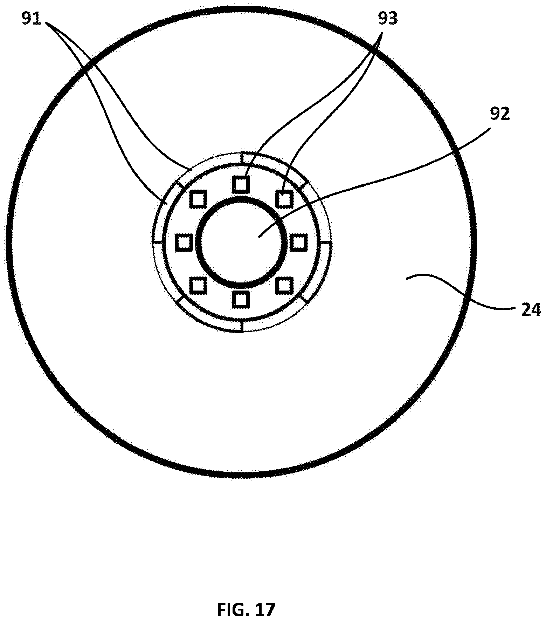

In some examples, the prime mover, in particular the electric motor 25, is integrated with the flywheel 24. In these examples, as shown in FIG. 17, the flywheel 24 has a plurality of permanent magnets 91 of alternating polarity mounted about an inner circumference. A stator 92 is provided within the inner circumference of the flywheel 24 and includes alternating field windings 93. In this arrangement, the flywheel 24 acts as the rotor of the motor and is caused to rotate by means of a correspondingly alternating polarity of the windings 93 in a conventional manner. Such an arrangement provides for a lighter weight, more compact rotatable flywheel assembly.

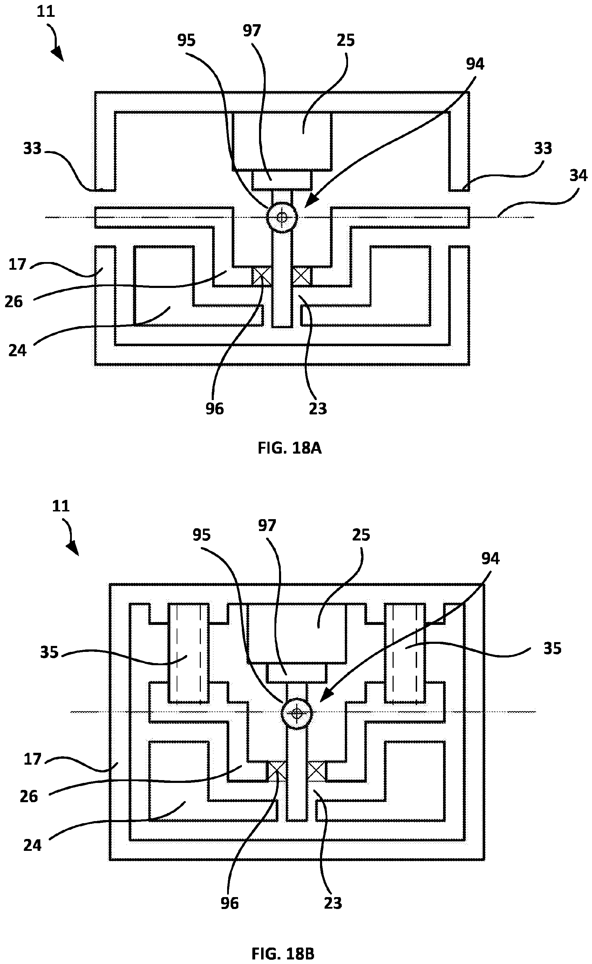

In other examples of the gyroscope device 11, the prime mover, in particular the motor 25, is not directly coupled to the flywheel 24. As shown in FIGS. 18A and 18B, which show perpendicular cross-sections of a gyroscope device 11, a transmission 94 is provided between the motor 25 and the flywheel 24 to transfer rotation from the motor 25 to the flywheel 24.

In this example, the motor 25 is fixed to the housing 17, and the transmission 94 comprises a flexible or articulated shaft 95 that accommodates precession of the flywheel 24 about the precession axis 34, relative to the motor 25. Such an arrangement advantageously means that the motor 25 does not need to be mounted for rotation about the precession axis 34, making flywheel 24 precession more reactive to lower amplitude/acceleration tremors as the mass of the rotatable flywheel assembly 23 is lower. In addition, electrical connection to the motor 25 is simplified as the motor 25 is not moving relative to the housing 17.

As shown, the articulated shaft 95 extends from the motor 25 to the flywheel 24 and the articulated shaft 95 can bend in the plane of the rotation about the precession axis 34 (in the plane of the page of FIG. 18B). The gimbal 26 is hingedly mounted to the housing 17 at hinge seats 33 in the same manner as described with reference to FIGS. 3A and 3B to define the precession axis 34. The articulated shaft 95 is rotatably mounted to the gimbal 26 at a bearing 96 so that the gimbal 26 and flywheel 24 are suspended on the articulated shaft 95. The articulated shaft 95 can bend at a position between the gimbal 26 and the motor 25. Therefore, the articulated shaft 95 permits precession of the gimbal 26 and the flywheel 24 about the precession axis 34. The gimbal 26 and biasing members function in the same manner as earlier examples, in particular examples of FIGS. 3A and 3B.

In some examples, the transmission 94 may further comprise a clutch 97 arranged between the motor 25 from the flywheel 24 and configured to disengage the rotational connection between the motor 25 and the flywheel 24. Advantageously, this allows the flywheel 24 to spin freely of the motor 25 when the clutch 97 is disengaged. The clutch 97 may be controlled to disengage when the user is not experiencing tremors or when the user is not performing a task. In addition, the clutch 97 may be configured to disengage when the gyroscope device 11 is taken off or dropped, thereby protecting the motor 25 from forces generated by the momentum of the flywheel 24 in such a situation.

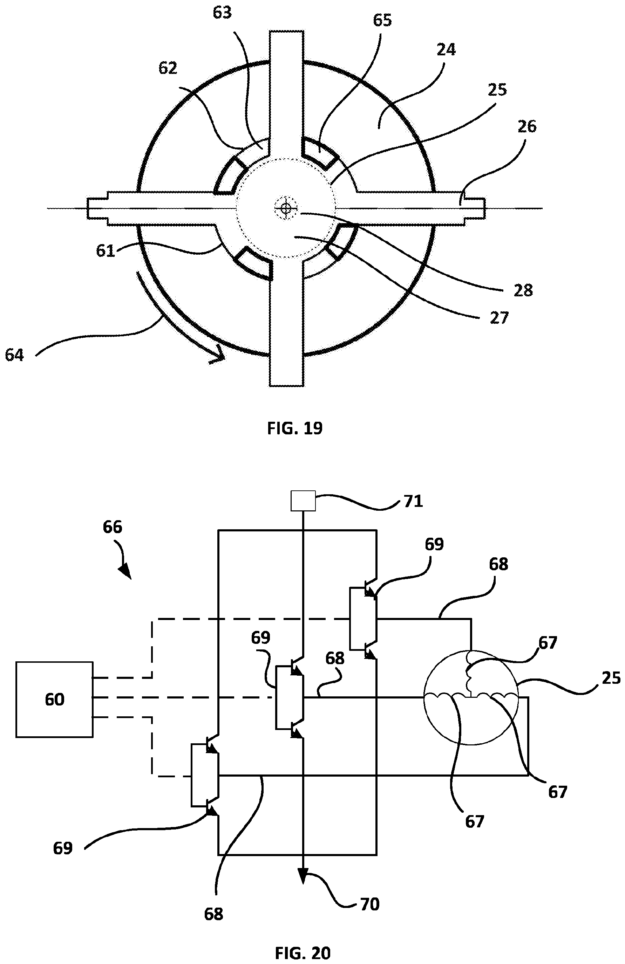

FIG. 19 schematically illustrates an example arrangement of the motor 25 and flywheel 24. In this example, the stator 27 of the motor 25 is mounted to the gimbal 26. The gimbal 26 comprises an opening or recess 61 in which the stator 27 is located, and the recess 61 includes a plurality of slots 62 formed in an inner face of the recess 61, adjacent to the stator 27. The slots are preferably arcuate. As shown, in this example the recess 61 includes four slots 62 distributed about the recess 61, preferably evenly distributed. In other examples, the recess 61 may have more or fewer slots, for example two, three or six slots 62. The stator 27 of the motor 25 comprises radial tabs 63 that extend from an outer circumferential face of the stator 27 and protrude into the slots 62. The rotor 28 of the motor 25 is arranged to rotate flywheel 24 in the direction of arrow 64. A spring 65 is arranged between each radial tab 63 and a side of the corresponding recess 62 on a side of the radial tab 63 that is opposite to the direction of rotation 64. In this way, the springs 65 are arranged to reduce the inertia transferred to the stator 27 as the motor 25 starts to rotate the flywheel 24, i.e. when motor 25 torque is highest. Preferably, for the gyroscope device 11 described hereinbefore, the flywheel 24 has a high inertia and the motor 25 is compact and low energy. The arrangement of the radial tabs 63 and springs 65 illustrated in FIG. 19 reduces transfer of inertia from the flywheel 24 to the gimbal 26 (and therefore the housing 17 of the gyroscope device 11) during start of the rotation of the flywheel 24, when torque is at a maximum. This makes the gyroscope device 11 more comfortable for a user to wear on their body when the gyroscope device 11 is started up.

In other examples, the slots 62 are formed in a motor housing that at least partly surrounds the stator 27, and the housing is in turn mounted to the gimbal 26.

FIG. 20 illustrates a motor control circuit 66 for the motor 25 of the gyroscope device 11. The motor control circuit 66 may be provided by the controller 60 described with reference to any of FIGS. 10, 11 and 12. The motor control circuit 66 comprises a power supply 68 for three windings 67 of the motor 25. Each power supply 68 comprises a switch 69, controllable by the controller 60 for switching between a drive configuration in which power is provided from the power source 71, for example a battery, to a winding 67 to drive the motor 25, and a braking configuration in which the winding 67 is shorted to earth 70. To brake the motor 25 to slow or stop the motor 25 and flywheel 24 rotation, the controller 50 configures all of the switches 69 to short the windings 67 to earth 70. In this configuration, the electromagnetic effects generated in the motor result in a braking effect on the motor 25 and flywheel 24. This can more quickly stop rotation of the flywheel 24 while also reducing torque felt by the user.

In preferred examples, the controller 50 is configured to brake the motor 25 in pulses by sequentially changing between a zero-power switch 69 configuration and an earthed switch 69 configuration. Pulsing the braking effect on the motor 25 reduces counter torques generated and experienced by the user wearing the gyroscope device 11.

In the configuration shown in FIG. 20 the motor 25 has three windings 67 but it will be appreciated that the motor 25 may have more windings, for example four windings 67, five windings 67, or more.

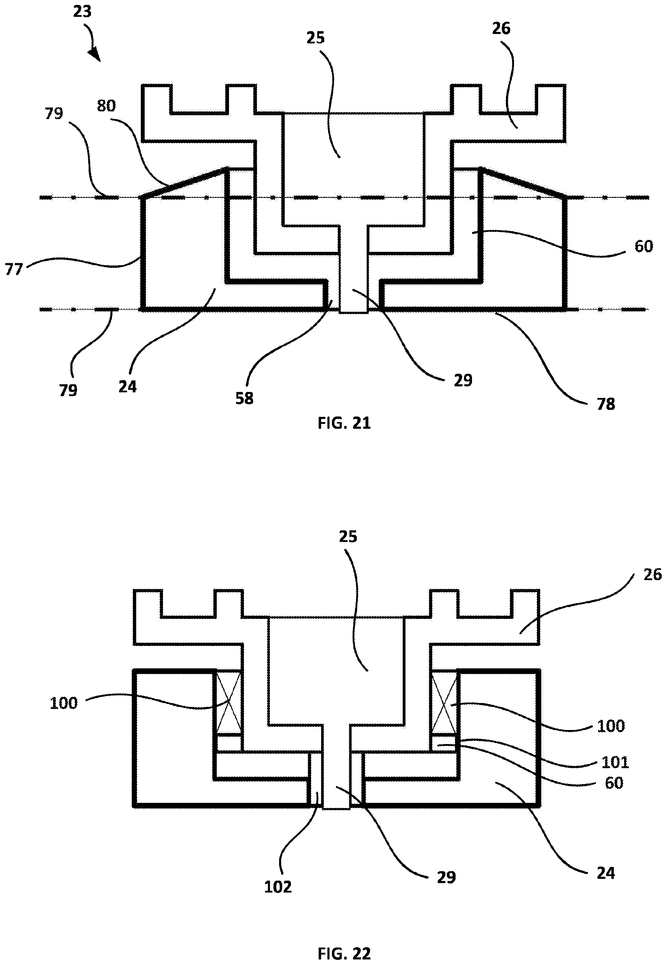

FIG. 21 shows rotatable flywheel assembly 23 for the gyroscope device 11, including a flywheel 24. In particular, FIG. 21 shows a rotatable flywheel assembly 23 the gyroscope device of FIGS. 3A and 3B. Preferably, the flywheel 24 is highly balanced to reduce vibrations and noise generated by rotation of the flywheel 24 at high speeds during operation of the gyroscope device 11. This is especially beneficial as the gyroscope device 11 is worn on a user's body, for example on the hand, during day-to-day activities where the generation of vibrations and noise are undesirable. A highly balanced flywheel 24 will also increase the operational life of the motor 25 and any bearings or other mounts (e.g. the hinge) in the gyroscope device 11. Protecting the bearings, or increasing their life, provides a more reliable and longer lasting gyroscope device.

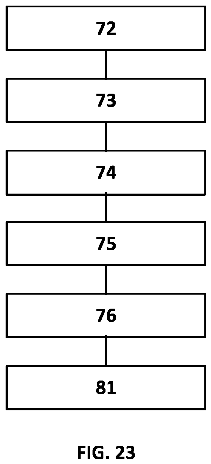

As shown in FIG. 21 and described previously, the flywheel 24 preferably comprises a flat face 78 and a recessed cavity 60 on a side of the flywheel 24 opposite to the flat face 78. As described below with reference to FIG. 23, the flywheel 24 is balanced on two planes 79.

As shown in FIG. 21 and other examples described previously, the flywheel 24 is mounted to the motor shaft 29. In these examples, the flywheel 24 is entirely supported by the motor shaft 29, which provides for low friction rotation of the flywheel 24. In a further example, illustrated in FIG. 22, a bearing 100 is provided between the flywheel 24 and the gimbal 26. The bearing 100 is arranged between an inner circumferential face 101 of the flywheel 24, within the recessed cavity 60, and the gimbal 26. The bearing 100 may be a rolling element bearing, for example a ball bearing or cylindrical roller bearing, or it may be a bushing.

The bearing 100 provides support for the flywheel 24 and help to reduce transfer of non-rotational forces between the flywheel 24 and the motor 25. For example, if the gyroscope device 11 were dropped then the impact momentum generated by the flywheel 24 will not be entirely imparted onto the motor shaft 29 as some of it will be imparted onto the gimbal 26 via the bearing 100, helping to protect the motor 25 from impact forces.

Additionally or alternatively, as illustrated in FIG. 22, a rubber insert 102 may be provided between the flywheel 24 and the motor shaft 29. This also helps to reduce transfer of non-rotational forces between the motor 25 and the flywheel 24 to help protect the motor 25 from impact forces. The rubber insert 102 is preferably thin and rigid so that torque transfer for rotation of the flywheel 24 is not significantly reduced.

FIG. 23 illustrates a method of manufacturing a flywheel 24 for the gyroscope device 11 of the tremor stabilisation apparatus. The flywheel 24 is preferably made of a metal, for example brass, and is manufactured from a cylindrical blank.

The manufacturing process comprises a first stage 72 of machining, on a lathe, from the cylindrical blank, the form of the flywheel 24. During machining 72 the lathe is used to turn the outer circumferential surface 77 of the flywheel 24, the upper surface 80 of the flywheel 24, the recessed cavity 60, and the motor mounting hole 58 from the direction of the upper surface 80. That is, the above surfaces and features of the flywheel 24 are machined from an end of the cylindrical blank protruding from a chuck of the lathe.

In a second stage 73 of the process, the flywheel 24 is cut from the cylindrical blank by cutting the face 78 perpendicular to the axis of rotation of the lathe to separate the flywheel from the cylindrical blank. Cutting the face 78 so that it is flat or convex means that the flywheel 24 can be completely machined in a single clamping operation, without having to re-clamp the flywheel 24 in the lathe, which may introduce an eccentricity.

Advantageously, by machining the flywheel 24 on the lathe from only the direction of the upper surface 80 and cutting the flywheel 24 from the blank, as per stage 73 described above, all of the surfaces of the flywheel 24 are machined without removing the flywheel 24 from the lathe. That is, the flywheel 24 is machined without re-clamping the material blank, which might introduce an eccentricity. This results in better tolerance between the surfaces of the flywheel 24, and reduces initial unbalance in the flywheel 24.

Next, in stage 74, the machined flywheel 24 is mounted to a motor 25 and a gimbal 26 of a gyroscope device 11 to form the rotatable flywheel assembly 23 described above, for example with reference to FIGS. 3A and 3B. In particular, the motor 25 is attached to the gimbal 26 and then the motor shaft 29 is press fitted onto the motor mounting hole 58 of the flywheel 24.

Subsequently, in stage 75, the rotatable flywheel assembly 23 is balanced to improve the balance of the rotatable flywheel assembly 23, in particular the flywheel 24. This stage 75 comprises mounting the rotatable flywheel assembly 23 to an accelerometer assembly comprising a mount for the gimbal 26, a plurality of accelerometers for detecting vibrations in the gimbal 26, and laser ablation apparatus for removing material from the flywheel 24 by laser ablation. The laser ablation apparatus is arranged to remove material from the flywheel 24 at planes 79 illustrated in FIG. 17. The two planes 79 are located at the edges of the circumferential surface 77 of the flywheel 24, adjacent the lower face 78 and the upper face 80. Removing material from the flywheel 24 at planes 79 provides the most effective form of balancing because mass removed from the circumferential face 77 of the flywheel 24 will have the greatest effect at reducing imbalance, and providing two planes 79 allows an acceptable balance grade to be achieved with less overall material removal, reducing any effect on the inertia and angular momentum provided by the flywheel 24 in operation.

In other examples, material can be removed from the flywheel 24 using other methods, for example mechanical drilling or cutting. Preferably, material is removed from the flywheel 24 by a non-contact operation that does not mechanically contact the flywheel 24, for example laser ablation or electron beam ablation. Advantageously, a non-contact operation does not cause vibrations in the flywheel 24 that might damage the motor 25. In other examples, material can be added to the flywheel 24 to balance the flywheel 24, for example by material deposition such as by welding additional material to the flywheel 24, or by drilling a hole in the flywheel 24 and inserted a heavier material in the hole. Preferably, material is added to the flywheel 24 by a non-contact material deposition operation, for example physical vapor deposition such as pulsed laser deposition.

In stage 76, after mounting the rotatable flywheel assembly 23 to the accelerometer assembly, the motor 25 of the rotatable flywheel assembly 23 is powered to rotate the flywheel 24 at a first speed, and material is removed from the flywheel 24 by laser ablation based on vibrations detected by the accelerometers to reduce vibrations caused by the flywheel 24. This improves the balance of the rotatable flywheel assembly 23.

Next, in stage 81, the motor 25 of the rotatable flywheel assembly 23 increases the rotational speed of the flywheel 24 to a second speed, greater than the first speed of stage 76, and material is removed from the flywheel 24 by laser ablation based on vibrations detected by the accelerometers to reduce vibrations caused by the flywheel 24.

Optionally, stage 81 is repeated at even higher rotational speeds than the second speed.

The above method provides a balanced rotatable flywheel assembly 23.

Advantageously, using the motor 25 of the gyroscope device 11 during the balancing process means that the rotatable flywheel assembly 23 (i.e. the flywheel 24, gimbal 26 and motor 25) are balanced as a single unit, which provides very accurate tolerances between the motor shaft 29 and the circumferential surface 77 of the flywheel 24. The balanced rotatable flywheel assembly 23 assembly can then be assembled into the gyroscope device 11 without disturbing the balance of the rotatable flywheel assembly 23. The rotatable flywheel assembly 23 is preferably not disassembled before being assembled into a gyroscope device 11, in particular the housing 17 as illustrated in FIGS. 3A and 3B.

Advantageously, performing balancing at a first speed, and then performing balancing at a second, higher speed, protects the motor 25, specifically the bearings of the motor 25, from vibrations generated by the initially unbalanced flywheel. This allows the same motor 25 to be used in the gyroscope device 11 without having disassemble the rotatable flywheel assembly 23 before being assembled into a gyroscope device 11.

The inventors have found that the above method of manufacturing and balancing the flywheel 24 has provided a balanced rotatable flywheel assembly 23 that exceeds the limits specified in ISO 1940/1, i.e. achieving a balance grade of lower than G0.4.

This high degree of balancing is particularly useful in the tremor stabilisation apparatus described herein as it minimises or eliminates vibrations and noise, which is beneficial to the user and also extends the operational life of the gyroscope device 11 and extends battery life because less power is required to drive the flywheel 24.

Although the apparatus of the present invention has been described primarily with respect to therapeutic benefits for suffers of neurological conditions inducing relatively strong tremors, the present invention is equally suitable for other uses where stabilisation of hand vibrations (for example), such as those at a normal level caused simply by pulsation of blood flow would be beneficial, such as in sports (such as archery, darts or golf); fine arts, such as painting fine detail; photography or in surgery.

For the avoidance of doubt, features or aspects of the present invention which are described herein with respect to a specific embodiment are not limited to that embodiment. The features described may be combined in any combination. Any and all such combinations are encompassed by the invention and shall not and do not constitute added subject matter.

## Claims

1. Tremor stabilisation apparatus (11) comprising a housing (17) that is attachable to a part of a user's body, and a rotatable flywheel assembly (23) mounted to the housing, the rotatable flywheel assembly comprising:
a rotatable flywheel (24);
a prime mover (25) arranged to rotate the flywheel about a flywheel rotation axis (38); and
a gimbal (26) to which the flywheel is attached, the gimbal being pivotally mounted to the housing at a hinge (32, 33) formed between the gimbal and the housing and defining a precession axis (34) such that the flywheel can process with respect to the housing about the precession axis,
wherein the tremor stabilisation apparatus further comprises a biasing member (35) arranged to oppose precession of the rotatable flywheel assembly and urge the rotatable flywheel assembly to an equilibrium position;
**characterised in that** the precession axis is fixed relative to the housing.

2. The tremor stabilisation apparatus of claim 1, wherein the housing (17) and the gimbal (26) are arranged to provide a maximum angle of precession about the precession axis (34) from the equilibrium position of about 60 degrees or less, preferably about 30 degrees or less, more preferably about 10 degrees, most preferably about 5 degrees.

3. The tremor stabilisation apparatus of claim 2, wherein the housing (17) comprises a stop arranged to contact the gimbal (26) when the gimbal is rotated to the maximum angle of precession.

4. The tremor stabilisation apparatus of claim 3, wherein the stop comprises a seat (41) arranged to support the biasing member (35).

5. The tremor stabilisation apparatus of any preceding claim, wherein the biasing member (35) is arranged between the gimbal (26) and the housing (17), and wherein the tremor stabilisation apparatus further comprises an elastomeric damper (42) disposed between the biasing member and the housing.

6. The tremor stabilisation apparatus of any preceding claim, wherein the biasing member (35) is configured to increase a biasing force provided by the biasing member as the angle of precession increases.

7. The tremor stabilisation apparatus of any of claims 1 to 6, wherein the biasing member (35) is arranged between the gimbal (26) and the housing (17), and wherein the biasing member comprises a magnetic assembly comprising:
a first magnet (98) attached to the gimbal, and
a second magnet (99) attached to the housing such that the first and second magnets repel each other.

8. The tremor stabilisation apparatus of any preceding claim, wherein a side of the housing (17) comprises a mount (15) for attachment of the tremor stabilisation apparatus to the part of the user's body, the side of the housing defining a plane that is approximately parallel to a surface of the part of the user's body, and wherein in the equilibrium position an angle between the flywheel rotation axis (38) and a normal line from the plane of the side of the housing is between about +45 degrees and about -45 degrees, preferably about 0 degrees.

9. The tremor stabilisation apparatus of any preceding claim, wherein the biasing member comprises an adjustable force biasing member (47) comprising an actuator (48) for adjusting the biasing force provided by the adjustable force biasing member, and wherein the tremor stabilisation apparatus further comprises a controller (50) arranged to control the actuator to adjust the biasing force.

10. The tremor stabilisation apparatus of claim 9, further comprising a sensor (49) arranged to detect a characteristic of a movement of the part of the user's body to which the tremor stabilisation apparatus is attached in use, and wherein the controller (50) is configured to receive a signal from the sensor and control the biasing force of the adjustable force biasing member (47) based on the detected characteristic.

11. The tremor stabilisation apparatus of any of claims 9 or claim 10, further comprising a sensor arranged to detect rotation of the flywheel (24) about the precession axis (34), and wherein the controller (50) is configured to receive a signal from the sensor and control the biasing force of the adjustable force biasing member (47) based on the detected rotation of the flywheel about the precession axis.

12. The tremor stabilisation apparatus of any preceding claim, further comprising:
a prime mover controller (60) configured to control the prime mover (25) and the rotational speed of the flywheel (24) about the flywheel rotation axis, and
a sensor arranged to detect one or both of:
a characteristic of a movement of the part of the user's body to which the tremor stabilisation apparatus is attached in use; and/or,
rotation of the flywheel about the precession axis;
wherein the prime mover controller is configured to receive a signal from the sensor or sensors and control the rotational speed of the flywheel (24) based on one or
both of: the detected characteristic of a movement of the part of the user's body; and rotation of the flywheel about the precession axis.

13. The tremor stabilisation apparatus of any preceding claim, wherein the gimbal (26) comprises a mounting portion for attachment of the flywheel (24), and a hinge portion (32) extending radially of the mounting portion to cooperate with a hinge seat (33) of the housing (17) to form the hinge defining the precession axis (34).

14. The tremor stabilisation apparatus of any of claims 1 to 13, wherein one of the gimbal (26) and the housing (17) comprises a ball (83), and the other of the gimbal and the housing comprises a socket (84) adapted to receive the ball, and wherein the ball is configured to rotate within the socket to define the precession axis (34).

## Patentansprüche

1. Vorrichtung zur Tremorstabilisierung (11), umfassend ein Gehäuse (17), das an einem Körperteil eines Benutzers anbringbar ist, und eine drehbare Schwungradanordnung (23), die an dem Gehäuse montiert ist, die drehbare Schwungradanordnung umfassend:
ein drehbares Schwungrad (24);
eine Antriebsmaschine (25), die angeordnet ist, um das Schwungrad um eine Schwungraddrehachse (38) zu drehen; und
einen Kardanring (26), an dem das Schwungrad angebracht ist, wobei der Kardanring an einem Gelenk (32, 33), das zwischen dem Kardanring und dem Gehäuse gebildet ist, schwenkbar an dem Gehäuse montiert ist und eine Präzessionsachse (34) definiert, sodass das Schwungrad in Bezug auf das Gehäuse um die Präzessionsachse präzedieren kann,
wobei die Vorrichtung zur Tremorstabilisierung ferner ein Vorspannelement (35) umfasst, das angeordnet ist, um einer Präzession der drehbaren Schwungradanordnung entgegenzuwirken und die drehbare Schwungradanordnung in eine Gleichgewichtsposition zu zwingen;
**dadurch gekennzeichnet, dass** die Präzessionsachse in Bezug auf das Gehäuse feststehend ist.

2. Vorrichtung zur Tremorstabilisierung nach Anspruch 1, wobei das Gehäuse (17) und der Kardanring (26) angeordnet sind, um einen maximalen Präzessionswinkel um die Präzessionsachse (34) aus der Gleichgewichtsposition von etwa 60 Grad oder weniger, vorzugsweise etwa 30 Grad oder weniger, bevorzugter etwa 10 Grad, am meisten bevorzugt etwa 5 Grad, bereitzustellen.

3. Vorrichtung zur Tremorstabilisierung nach Anspruch 2, wobei das Gehäuse (17) einen Anschlag umfasst, der angeordnet ist, um den Kardanring (26) zu berühren, wenn der Kardanring auf den maximalen Präzessionswinkel gedreht wird.

4. Vorrichtung zur Tremorstabilisierung nach Anspruch 3, wobei der Anschlag einen Sitz (41) umfasst, der angeordnet ist, um das Vorspannelement (35) zu tragen.

5. Vorrichtung zur Tremorstabilisierung nach einem der vorhergehenden Ansprüche, wobei das Vorspannelement (35) zwischen dem Kardanring (26) und dem Gehäuse (17) angeordnet ist, und wobei die Vorrichtung zur Tremorstabilisierung ferner eine elastomere Klemmvorrichtung (42) umfasst, die zwischen dem Vorspannelement und dem Gehäuse angeordnet ist.

6. Vorrichtung zur Tremorstabilisierung nach einem der vorhergehenden Ansprüche, wobei das Vorspannelement (35) konfiguriert ist, um mit zunehmendem Präzessionswinkel eine Vorspannkraft zu erhöhen, die von dem Vorspannelement bereitgestellt wird.

7. Vorrichtung zur Tremorstabilisierung nach einem der Ansprüche 1 bis 6, wobei das Vorspannelement (35) zwischen dem Kardanring (26) und dem Gehäuse (17) angeordnet ist, und wobei das Vorspannelement eine magnetische Anordnung umfasst, umfassend:
einen ersten Magneten (98), der an dem Kardanring angebracht ist, und
einen zweiten Magneten (99), der an dem Gehäuse angebracht ist, sodass sich der erste und der zweite Magnet gegenseitig abstoßen.

8. Vorrichtung zur Tremorstabilisierung nach einem der vorhergehenden Ansprüche, wobei eine Seite des Gehäuses (17) eine Halterung (15) zum Anbringen der Vorrichtung zur Tremorstabilisierung an dem Körperteil des Benutzers umfasst, wobei die Seite des Gehäuses eine Ebene definiert, die ungefähr parallel zu einer Oberfläche des Körperteils des Benutzers ist, und wobei in der Gleichgewichtsposition ein Winkel zwischen der Schwungraddrehachse (38) und einer Normalen von der Ebene der Seite des Gehäuses zwischen etwa +45 Grad und etwa -45 Grad, vorzugsweise etwa 0 Grad, ist.

9. Vorrichtung zur Tremorstabilisierung nach einem der vorhergehenden Ansprüche, wobei das Vorspannelement ein Vorspannelement einstellbarer Kraft (47) umfasst, umfassend einen Aktuator (48) zum Einstellen der von dem Vorspannelement einstellbarer Kraft bereitgestellten Vorspannkraft, und wobei die Vorrichtung zur Tremorstabilisierung ferner eine Steuerung (50) umfasst, die angeordnet ist, um den Aktuator zu steuern, um die Vorspannkraft einzustellen.

10. Vorrichtung zur Tremorstabilisierung nach Anspruch 9, ferner umfassend einen Sensor (49), der angeordnet ist, um ein Merkmal einer Bewegung des Körperteils des Benutzers zu erfassen, an dem die Vorrichtung zur Tremorstabilisierung im Gebrauch angebracht ist, und wobei die Steuerung (50) konfiguriert ist, um ein Signal von dem Sensor zu empfangen und die Vorspannkraft des Vorspannelements einstellbarer Kraft (47) basierend auf dem erfassten Merkmal zu steuern.

11. Vorrichtung zur Tremorstabilisierung nach einem der Ansprüche 9 oder 10, ferner umfassend einen Sensor, der angeordnet ist, um eine Drehung des Schwungrads (24) um die Präzessionsachse (34) zu erfassen, und wobei die Steuerung (50) konfiguriert ist, um ein Signal von dem Sensor zu empfangen und die Vorspannkraft des Vorspannelements einstellbarer Kraft (47) basierend auf der erfassten Drehung des Schwungrads um die Präzessionsachse zu steuern.

12. Vorrichtung zur Tremorstabilisierung nach einem der vorhergehenden Ansprüche, ferner umfassend:
eine Antriebsmaschinensteuerung (60), die konfiguriert ist, um die Antriebsmaschine (25) und die Drehgeschwindigkeit des Schwungrads (24) um die Schwungraddrehachse zu steuern, und
einen Sensor, der angeordnet ist, um eines oder beide von Folgenden zu erfassen:
ein Merkmal einer Bewegung des Körperteils des Benutzers, an dem die Vorrichtung zur Tremorstabilisierung im Gebrauch angebracht ist; und/oder,
Drehung des Schwungrads um die Präzessionsachse;
wobei die Antriebsmaschinensteuerung konfiguriert ist, um ein Signal von dem Sensor oder den Sensoren zu empfangen und die Drehgeschwindigkeit des Schwungrads (24) basierend auf einem oder beiden von Folgenden zu steuern: dem erfassten Merkmal einer Bewegung des Körperteils des Benutzers; und einer Drehung des Schwungrads um die Präzessionsachse.

13. Vorrichtung zur Tremorstabilisierung nach einem der vorhergehenden Ansprüche, wobei der Kardanring (26) einen Montageabschnitt zum Anbringen des Schwungrads (24) und einen Gelenkabschnitt (32) umfasst, der sich radial von dem Montageabschnitt erstreckt, um mit einem Gelenksitz (33) des Gehäuses (17) zusammenzuwirken und das Gelenk zu bilden, das die Präzessionsachse (34) definiert.

14. Vorrichtung zur Tremorstabilisierung nach einem der Ansprüche 1 bis 13, wobei eines von dem Kardanring (26) und dem Gehäuse (17) eine Kugel (83) umfasst und der/das andere von dem Kardanring oder dem Gehäuse, eine Fassung (84) umfasst, die angepasst ist, um die Kugel aufzunehmen, und wobei die Kugel konfiguriert ist, um in der Fassung zu drehen, um die Präzessionsachse (34) zu definieren.

## Revendications

1. Appareil de stabilisation de tremblements (11) comprenant un boîtier (17) qui peut être fixé à une partie du corps d'un utilisateur, et un ensemble volant rotatif (23) monté sur le boîtier, l'ensemble volant rotatif comprenant :
un volant rotatif (24) ;
un moteur d'entraînement (25) agencé pour faire tourner le volant autour d'un axe de rotation du volant (38) ; et
un cardan (26) auquel le volant est fixé, le cardan étant monté pivotant sur le boîtier au niveau d'une charnière (32, 33) formée entre le cardan et le boîtier et définissant un axe de précession (34) de sorte que le volant puisse être animé d'un mouvement de précession par rapport au boîtier autour de l'axe de précession,
ledit appareil de stabilisation de tremblements comprenant en outre un élément de sollicitation (35) agencé pour s'opposer à la précession de l'ensemble volant rotatif et pousser l'ensemble volant rotatif vers une position d'équilibre ;
**caractérisé en ce que** l'axe de précession est fixe par rapport au boîtier.

2. Appareil de stabilisation de tremblements selon la revendication 1, ledit boîtier (17) et ledit cardan (26) étant agencés pour fournir un angle de précession maximal autour de l'axe de précession (34) à partir de la position d'équilibre d'environ 60 degrés ou moins, de préférence d'environ 30 degrés ou moins, plus préférablement d'environ 10 degrés, mieux encore d'environ 5 degrés.

3. Appareil de stabilisation de tremblements selon la revendication 2, ledit boîtier (17) comprenant une butée agencée pour entrer en contact avec le cardan (26) lorsque le cardan tourne jusqu'à l'angle de précession maximal.

4. Appareil de stabilisation de tremblements selon la revendication 3, ladite butée comprenant une assise (41) agencée pour supporter l'élément de sollicitation (35).

5. Appareil de stabilisation de tremblements selon l'une quelconque des revendications précédentes, ledit élément de sollicitation (35) étant disposé entre le cardan (26) et le boîtier (17), et ledit appareil de stabilisation de tremblements comprenant en outre un dispositif de serrage en élastomère (42) disposé entre l'élément de sollicitation et le boîtier.

6. Appareil de stabilisation de tremblements selon l'une quelconque des revendications précédentes, ledit élément de sollicitation (35) étant conçu pour augmenter une force de sollicitation délivrée par l'élément de sollicitation à mesure que l'angle de précession augmente.

7. Appareil de stabilisation de tremblements selon l'une quelconque des revendications 1 à 6, ledit élément de sollicitation (35) étant disposé entre le cardan (26) et le boîtier (17), et ledit élément de sollicitation comprenant un ensemble magnétique comprenant :
un premier aimant (98) fixé au cardan, et
un second aimant (99) fixé au boîtier de sorte que les premier et second aimants se repoussent l'un l'autre.

8. Appareil de stabilisation de tremblements selon l'une quelconque des revendications précédentes, un côté du boîtier (17) comprenant un support (15) destiné à fixer l'appareil de stabilisation de tremblements à la partie du corps de l'utilisateur, le côté du boîtier définissant un plan qui est approximativement parallèle à une surface de la partie du corps de l'utilisateur, et dans ladite position d'équilibre, l'angle entre l'axe de rotation du volant (38) et une ligne normale partant du plan du côté du boîtier étant compris entre environ +45 degrés et environ -45 degrés, de préférence d'environ 0 degré.

9. Appareil de stabilisation de tremblements selon l'une quelconque des revendications précédentes, ledit élément de sollicitation comprenant un élément de sollicitation à force ajustable (47) comprenant un actionneur (48) destiné à ajuster la force de sollicitation délivrée par l'élément de sollicitation à force ajustable, et ledit appareil de stabilisation de tremblements comprenant en outre un dispositif de commande (50) agencé pour commander l'actionneur afin d'ajuster la force de sollicitation.

10. Appareil de stabilisation de tremblements selon la revendication 9, comprenant en outre un capteur (49) agencé pour détecter une caractéristique d'un mouvement de la partie du corps de l'utilisateur à laquelle l'appareil de stabilisation de tremblements est fixé lors de l'utilisation, et ledit dispositif de commande (50) étant configuré pour recevoir un signal provenant du capteur et commander la force de sollicitation de l'élément de sollicitation à force ajustable (47) en fonction de la caractéristique détectée.

11. Appareil de stabilisation de tremblements selon l'une quelconque des revendications 9 ou 10, comprenant en outre un capteur agencé pour détecter la rotation du volant (24) autour de l'axe de précession (34), et ledit dispositif de commande (50) étant configuré pour recevoir un signal du capteur et commander la force de sollicitation de l'élément de sollicitation à force ajustable (47) sur la base de la rotation détectée du volant autour de l'axe de précession.

12. Appareil de stabilisation de tremblements selon l'une quelconque des revendications précédentes, comprenant en outre :
un dispositif de commande de moteur d'entraînement (60) configuré pour commander le moteur d'entraînement (25) et la vitesse de rotation du volant (24) autour de l'axe de rotation du volant, et
un capteur agencé pour détecter l'une ou les deux parmi :
une caractéristique d'un mouvement de la partie du corps de l'utilisateur à laquelle l'appareil de stabilisation de tremblements est fixé lors de l'utilisation ; et/ou,
la rotation du volant autour de l'axe de précession ;
ledit dispositif de commande de moteur d'entraînement étant configuré pour recevoir un signal provenant du ou des capteurs et commander la vitesse de rotation du volant (24) en fonction de l'une ou les deux parmi : la caractéristique détectée d'un mouvement de la partie du corps de l'utilisateur ; et la rotation du volant autour de l'axe de précession.

13. Appareil de stabilisation de tremblements selon l'une quelconque des revendications précédentes, ledit cardan (26) comprenant une partie support pour la fixation du volant (24), et une partie charnière (32) s'étendant radialement par rapport à la partie support de manière à coopérer avec un socle de charnière (33) du boîtier (17) afin de former la charnière définissant l'axe de précession (34).

14. Appareil de stabilisation de tremblements selon l'une quelconque des revendications 1 à 13, un élément parmi le cardan (26) et le boîtier (17) comprenant une bille (83), et l'autre élément parmi le cardan et le boîtier comprenant une douille (84) adaptée à la réception de la bille, et ladite bille étant conçue pour tourner à l'intérieur de la douille de manière à définir l'axe de précession (34).
